# EUROPEAN PATENT APPLICATION

(11) **EP 4 843 572 A1**
(43) Date of publication of application: **25.11.2026**
(21) Application number: 24918385.6
(22) Date of filing: 17.01.2024
(51) Int. Cl.: A61K 31/4196, A61K 47/34, A61P 35/00

(54) **IMPROVED AROMATASE INHIBITOR DOSING SCHEDULES AND COMPOSITIONS**

(71) Applicant: Laboratorios Farmacéuticos Rovi, S.A., 28037 Madrid (ES)
(72) Inventor: GUTIERRO ADURIZ, Ibon, 28037 Madrid (ES); FRANCO RODRIGUEZ, Guillermo, 28037 Madrid (ES)
(74) Representative: Elzaburu S.L.P.
(86) International application number: PCT/ES2024/070030
(87) International publication number: WO 2025/153747

(57) **Abstract**

Improved formulations and dosing regimens are provided for the intramuscular administration of compositions containing aromatase inhibitors such as letrozole for use in the treatment of letrozole-responsive diseases, conditions or disorders. In the dosing regimens, a first dose of a long-acting injectable formulation is administered, and after a delay period, a second dose of a long-acting injectable formulation is administered, thereby providing overlapping dosing periods and enhancing oestrogen suppression.

## Description

### FIELD OF THE INVENTION

The present invention provides improved compositions and dosing schedules for intramuscular administration of aromatase inhibitor-containing compositions such as letrozole for use in the treatment of letrozole-responsive diseases, conditions, or disorders. Two doses of long-acting injectable (LAI) composition are administered sequentially to provide superior control of aromatase inhibitor plasma concentration and superior of suppression of estrogen. The LAI comprises a sterile biodegradable thermoplastic polymer of polylactic acid (PLA) and a solvent for the polymer. The LAI forms an intramuscular implant *in situ* at the site of administration, and the implant releases about 0.1-2 milligrams of aromatase inhibitor daily after administration to a subject in need thereof.

### BACKGROUND OF THE INVENTION

Improved cancer treatments need to be developed, not only new molecular entities but also pharmacological products for improving patients' quality of life. The development of prolonged release composition signifies an advance because they enable reducing the total dose administered, increasing the duration of each dose and the number of administrations and thereby create a positive impact on the emotional state of the patient.

Letrozole (4,4'-(1h,2,4-triazol-1-yl)methyl)dibenzonitrile) and anastrozole (2,2'-[5-(1 H-1,2,4-triazol-1-yl methyl )-1,3-phenylene ]bis(2-methylpropanenitrile)) belong to a class of drugs called nonsteroidal inhibitors of aromatase and their mechanism of action consists of reducing the amount of estrogen in the body. This effect can decelerate or stop the growth of many types of cancer-producing cells in the breast that need estrogen to grow.

Currently there is no formulation of letrozole on the market with the ability to control the release of the drug over a long period of time. The pharmaceutical drug letrozole is currently only available in tablet form for daily oral administration (FEMARA^{®}).

In the treatment of breast cancer, as in the treatment of cancer in general, the psychological state of the patient is very important; therefore, the development of a three-monthly formulation of letrozole and/or anastrozole would result in a substantial improvement in their quality of life, reducing the impact that would result from daily treatment. In turn, medical examinations that are carried out during monitoring of the disease are normally conducted at 3 and 6 months over the first few years, so the administration of the formulation could coincide with consultancy visits to the doctor.

Similar reasoning has led to the appearance on the market of formulations such as ZOLADEX^{®}, a preformed implant of goserelin for subcutaneous three-monthly application for the treatment of prostate carcinoma, and IMPLANON^{®}, a preformed implant of etonogestrel used as a contraceptive. However, these preformed implants show a series of disadvantages including:
- The preparation of the implants by extrusion requires the use of high temperatures, which can cause the degradation of the active ingredient and the generation of potentially toxic impurities;
- Low homogeneity of the product obtained when including active ingredients at low doses;
- Need for surgical procedures for implanting or injection of the implant using large diameter needles.

Literature also discloses implantable compositions of letrozole and/or anastrozole. For example, WO 2008/041245 A2 published April 10, 2008 to Panacea Biotec LTD describes implantable compositions comprising a wide variety of active ingredients such as some aromatase inhibitors, including anastrozole, in a wide variety of administration forms from preformed microparticles suspended in an aqueous vehicle to formulations that jellify *in situ*. Although it is doubtful that this document can sufficiently support all the combinations of active ingredients and administration forms that may arise, the examples always refer to preformed microparticles, that is it never describes systems of forming implants directly "*in situ*". Finally, it should be pointed out that none of the examples show a duration of over 60 days.

WO 2010/065358 A1 published June 10, 2010 to Glaser describes compositions for the administration of medicines containing testosterone and an aromatase inhibitor for continuous administration of testosterone and for preventing its conversion to estradiol. Although the description considers the possibility that the form of administration may be an implant, the only example of a form of administration is pellets.

Also, WO 2012/074883 A1 published June 7, 2012 to Durect Corp. describes biodegradable compositions for administration of pharmaceutical drugs. These compositions require the use of water-insoluble solvents such as benzyl benzoate or benzyl alcohol in order to maintain the implant in a liquid or semi-solid state. These solvents have been previously shown to provide sudden releases and therefore are not suitable for the prolonged release compositions of the present invention.

US 2008/0206303 A1 to Astra Zeneca describes prolonged release compositions of anastrozole comprising a PLA or PLGA polymer that can be accompanied by a wide variety of solvents; however, in the embodiments of the invention, the solvents used are benzyl alcohol and N-methyl-2-pyrrolidone (NMP), solvents that give rise to a very large burst followed by a subsequent almost zero release. In fact, the burst that was acceptable for the inventors in this document was 25-30% in one day, a very high value, and because of this none of their examples lasted more than 60 days; in particular in dogs, animals similar to humans, release did not continue longer than 35 days. Finally, no mention was made in this document of letrozole particle size nor of the importance of this factor in the behavior of the formulation.

Injectable compositions are disclosed by Jain et al. ("Injectable formulations of poly(lactic acid) and its copolymers in clinical use" in Adv. Drug Deliv. Rev. (2016), 107(14), 213-227), Kranz et al. ("A novel in situ forming drug delivery system for controlled parenteral drug delivery" in Inter. J. Pharma.

(2007), 332(1-2), 107-114, D'Souza et al. ("Methods to assess in vitro drug release from injectable polymeric particulate systems" in Pharm. Res. (2006), 23(3), 460-474), Li et al. ("Microfluidic fabrication of microparticles for biomedical applications" in Chem. Soc. Rev. (2018), 47(15), 5646-5683), Bassyouni et al. ("Advances and new technologies applied in controlled drug delivery system" in Res. Chem. Interm. (2013), 41(4), 2165-2200), Kissel et al. ("Parenteral depot-systems on the basis of biodegradable polymers" in J. Contr. Rel. (1991), 16(1-2), 27-41), Gomathi et al. ("Fabrication of letrozole formulation using chitosan nanoparticles through ionic gelation method" in Inter. J. Biol. Macromol. (2017), 104(7), 1820-1832), and Muralidhar et al. ("Controlled release injectable drug delivery: an overview" in J. Biomat. Res. (2017), 3(1), 6-15).

Injectable compositions are also disclosed in WO 2014/019972A1, ES 2390439A1, US 10285936B2, US 2019/0231682A1, US 2019/0365643A1, WO 2011/151356A2, EP 2394663A1, US 10058504B2, US 2019/0151230A1, US 2021/0154302 A1, US 2021/0168778 A1, WO 2019/238740 A1, US 10195138B2, US 10933015B2, and US 10912735B2 to Laboratorios Farmacéuticos ROVI, S.A. and WO2009060473A2 to Panacea Biotec Limited.

None of the art of record discloses sequential intramuscular administration of two or more doses of LAI composition as described and claimed herein, and none of the prior art recognizes the unexpected advantage that such a dosing regimen provides on control of letrozole plasma concentration and consequently on estrogen plasma concentration, i.e. estrogen suppression.

### SUMMARY OF THE INVENTION

The present invention seeks to overcome one or more of the disadvantages of known methods of using LAI compositions for the treatment of cancer, in particular for the adjuvant treatment of postmenopausal women with hormone receptor-positive advanced breast cancer or with locally advanced cancer or with metastatic cancer. Additional indications disclosed herein are also contemplated.

The present invention provides improved estrogen inhibition over a one-year period as compared to the inhibition provided by daily oral administration of 2.5 mg of letrozole (e.g. in FEMARA^{®}) and as compared to administration of a single LAI composition. In some embodiments, the dose of aromatase inhibitor in the LAI is about 50 mg or more, about 75 mg or more, or about 100 mg or more.

The invention provides dosing regimens for intramuscular administration of drug, e.g. letrozole and/or anastrozole, to a subject in need thereof. Throughout this document, all references to methods of drug administration or dosing regimens for drug administration should be interpreted as also referring to the compounds, compositions, and medicinal products intended for use in such regimens or methods.

A first aspect of the invention provides provides a long-acting injectable composition comprising polylactic acid (PLA), DMSO, and at least one aromatase inhibitor drug selected from the group consisting of letrozole, anastrozole, and combinations thereof, characterized in that the particle size distribution of the PLA is as follows:
- a mass particle size distribution with no more than 10% of particles larger than 300 microns and no more than 80% of particles smaller than 125 microns, when measured by analytical sieving according to USP<786>; and
- a particle size distribution by volume in which less than 10% of the particles are smaller than 20 microns, less than 10% of the particles are larger than 350 microns, the D50 is between 70 and 200 microns, the D80 is not less than 135 microns, and the D90 is not greater than 330 microns, when measured by laser diffraction analysis.

A second aspect of the invention provides a composition as described above for use in treating a subject in need thereof, and in particular in the treatment of a disease, condition, or disorder responsive to an aromatase inhibitor in the subject, comprising administering to the subject the aforementioned composition according to a dosing regimen comprising a) intramuscularly administering to a subject an amount of a long-acting injectable composition comprising a first dose of an aromatase inhibitor; and b) subsequently, after a delay period, administering intramuscularly an amount of a long-acting injectable composition comprising a second dose of an aromatase inhibitor, wherein the delay period is approximately 6 weeks or more after administering said first dose of the composition. In some embodiments, a) the amount of drug in the first dose is approximately the same as the amount of drug in the second dose; b) the amount of drug in the first dose is less than the amount of drug in the second dose; or c) the amount of drug in the first dose is greater than the amount of drug in the second dose.

For subjects needing continued treatment with aromatase inhibitor, the invention further comprises intramuscular administration of a third dose of the LAI composition at about 50-60 weeks, about 50-112 weeks, about 50-60 weeks, about 50 weeks, about 51 weeks, about 52 weeks, about 53 weeks, or about 54 weeks after administration of a prior dose of LAI composition. In some embodiments, the third dose of LAI composition is administered about 48-60 weeks after administration of the first LAI composition or about 48-60 weeks after administration of the second LAI composition. In some embodiments, the subject receives the first two doses of LAI composition but does not receive a third dose of LAI composition during the treatment period. In other embodiments, the subject receives a dose of LAI composition on an about annual basis after the first two doses during a treatment period.

The LAI composition may be administered to adipose tissue or muscular tissue. In preferred embodiments, the LAI composition is administered intramuscularly.

In some embodiments, the treatment period ranges from about 1 to 10 years, or is about 1 year or more, about 2 years or more, about 2 to 7 years, about 2 to 5 years, about 2 years, about 3 years, about 4 years, about 5 years, about 6 years, about 7 years, about 8 years, about 9 years, or about 10 years.

In some embodiments, a subject will experience remission, regression, or at least lack of progression of cancer following administration of a single dose of LAI composition. In other embodiments, a subject will experience remission, regression, or at least lack of progression of cancer following administration of two or more doses of LAI composition, wherein each dose is administered on an about annual basis.

The amount of aromatase inhibitor in the subsequent dose may be the same as, less than, or greater than the amount of aromatase inhibitor in the prior dose of LAI composition. In some embodiments of the first aspect of the invention, the amount of aromatase inhibitor in both LAI compositions is about the same and can be about 100 mg or more of aromatase inhibitor per dose.

In some embodiments of the first aspect of the invention, a non-long-acting injectable (nLAI) composition is administered in place of the first dose of LAI composition. For example, a subject is administered a dose of nLAI composition and about 6-10 weeks later, about 7-9 weeks later, or about 8 weeks later, the subject is administered a first dose of LAI composition. The nLAI composition comprises at least one pharmaceutical excipient, and drug, which is letrozole and/or anastrozole.

The doses of LAI composition are administered sequentially, meaning a first dose of LAI composition is administered first and after a delay period a second dose of LAI composition is administered. The first dose of LAI composition provides therapeutically effective plasma levels of drug for a first dosing period, and the second dose of LAI composition provides therapeutically effective plasma levels of drug for a second dosing period. For the first aspect of the invention, the beginnings of the respective dosing periods are offset by about 6-10 weeks, about 7-9 weeks, or about 8 weeks, and each respective dosing period lasts about 48-60 weeks, about 48-56 weeks, about 50-54 weeks, or about 52 weeks. For the second aspect of the invention, the beginnings of the respective dosing periods are offset by about 48-60 weeks, about 48-56 weeks, about 50-54 weeks, or about 52 weeks, and each respective dosing period lasts about 48-60 weeks, about 48-56 weeks, about 50-54 weeks, or about 52 weeks.

If a nLAI composition is administered, the nLAI composition provides a dosing period (period throughout which a therapeutic plasma concentration drug is present in a subject) of at least 6 weeks, at least 7 weeks or at least 8 weeks. The nLAI is administered before the LAI composition. The LAI composition is administered toward the end of or at the end of the dosing period of the nLAI. The LAI composition may be administered during the final 2 weeks of, the final week of, at completion of, or after completion of the dosing period of the nLAI composition. In some embodiments, the dosing period of the nLAI composition is about 7-9 weeks or about 8 weeks, and the LAI composition is administered about 7 weeks, about 8 weeks, or about 9 weeks after administration of the nLAI composition.

As compared to administration of a single dose of LAI composition on its own, the dosing regimens of the invention, i.e. sequential administration of at least two doses of LAI composition wherein their respective about 1-year dosing periods overlap and are offset by about 6-10 weeks or about 8 weeks (first aspect of the invention) or offset by about 50-54 weeks or about 52 weeks (second aspect of the invention), provides improved control of the plasma concentration of drug in the period after administration. The dosing regimens of the invention thereby provides improved E2 suppression and ultimately improved therapeutic efficacy.

The invention also provides a method of administering letrozole or anastrozole to a subject. The method comprises administering a first amount (or volume) of LAI composition comprising aromatase inhibitor and subsequently (after a delay period) administering a second amount (or volume) of LAI composition comprising aromatase inhibitor. The delay period can be in the range of about 6-10 weeks, about 7-9 weeks, or about 8 weeks for the first aspect of the invention and in the range of about 48-60 weeks, about 50-54 weeks, or about 52 weeks for the second aspect of the invention.

The invention also provides a method of administering drug, which is letrozole and/or anastrozole, to a subject. In some embodiments of the first aspect of the invention, the method comprises a) providing a first drug-containing LAI composition comprising drug; b) administering a first dose of said LAI composition to a subject in need thereof; c) providing a second drug-containing LAI composition; d) after about 6 weeks, about 7 weeks, about 8 weeks, or about 9 weeks of administering said first dose, administering a dose of said second drug-containing LAI composition to the subject. In some embodiments of the second aspect of the invention, the method comprises a) providing a first drug-containing LAI composition comprising drug; b) administering a first dose of said LAI composition to a subject in need thereof; c) providing a second drug-containing LAI composition; d) after about 48-60 weeks, about 50-54 weeks, or about 52 weeks of administering said first dose, administering a dose of said second drug-containing LAI composition to the subject. In some embodiments, the method can further comprise administering another dose of the LAI composition to the subject about 50-54 weeks or about 52 weeks after administration of the prior dose of LAI composition. The method of administration can be separate from a method of treatment; on the other hand, the method of administration may also be part of a method of treatment. The release of drug from the implant (a sustained (extended) release composition) may be as specified herein. In some embodiments, the LAI composition comprises (consists essentially of or consists of) drug, DMSO, and PLA.

Following administration, the LAI composition can form one or more implants. The composition can be administered to adipose tissue, muscle tissue, below the skin, or in the peritoneum.

In some embodiments, a) the amount of drug in the first dose approximates the amount of drug in the second dose; b) the amount of drug in the first dose is less than the amount of drug in the second dose; or c) the amount of drug in the first dose is greater than the amount of drug in the second dose. In some embodiments, the amount of drug in doses subsequent to the first and second doses a) approximates the amount of drug in the second dose; b) is less than the amount of drug in the second dose; or c) is greater than the amount of drug in the second dose.

The first aspect of the invention also provides a method of administering drug, which is letrozole and/or anastrozole, to a subject, the method comprising a) intramuscularly administering a first dose of drug-containing LAI composition; and b) subsequently, within a delay period of about 6-10 weeks, intramuscularly administering a second dose of drug-containing LAI composition. The amount of drug in a dose of LAI composition is independently selected at each occurrence from the group consisting of about 50-450 mg, about 50-100 mg, about 75 mg, about 80-150 mg, about 150-250 mg, about 350-450, about 100 mg, about 200 mg, or about 400 mg. The second dose of LAI composition can be administered about 6-10 weeks, about 7-9 weeks, or about 8 weeks after administration of the first dose of LAI composition.

The second aspect of the invention provides a method of administering drug, which is letrozole and/or anastrozole, to a subject, the method comprising a) intramuscularly administering a higher first dose of drug-containing LAI composition; and b) subsequently, after a delay period of about 48-60 weeks, intramuscularly administering a lower second dose of drug-containing LAI composition. The amount of drug in the first dose of LAI composition is about 200-400 mg, and the amount of drug in the second dose of LAI composition is about 75-100 mg. The second dose of LAI composition can be administered about 50-54 weeks, or about 52 weeks after administration of the first dose of LAI composition.

The methods of administering can be employed in or employed as methods of treating a letrozole-responsive or anastrozole-responsive disease, condition, or disorder.

The first aspect of the invention thus provides compositions and methods of treating an aromatase inhibitor responsive disease, condition, or disorder in a subject, the method comprising administering an amount (or volume) of LAI composition comprising aromatase inhibitor and subsequently (after a delay period) administering an amount (or volume) of LAI composition comprising aromatase inhibitor, wherein the delay period is about 6-10 weeks.

The second aspect of the invention provides compositions and methods of treating an aromatase inhibitor responsive disease, condition, or disorder in a subject, the method comprising administering an amount (or volume) of LAI composition comprising a higher first dose of aromatase inhibitor and subsequently (after a delay period) administering an amount (or volume) of LAI composition comprising a lower second dose of aromatase inhibitor, wherein the delay period is about 50-54 weeks or about 52 weeks.

The administering can be by injection. In some embodiments, the LAI compositions each have a dosing period of about 50-54 weeks, about 52 weeks, or about 1 year.

The method of administration and method of treatment of the invention employ a dosing regimen comprising a) providing LAI composition comprising aromatase inhibitor, wherein said LAI composition provides a dosing period of about 1 year; b) administering a first dose of said LAI composition; and c) after a delay period of about 6-10 weeks or about 50-54 weeks from said administering, administering a second dose of said LAI composition. The method optionally further comprises administering another dose of said LAI composition about 1 year after said first dose or said second dose. The method optionally further comprises administering a dose of said LAI composition annually for a total treatment period of about three years or more, about 5 years or more, about 7 years or more, or about 10 years. Intramuscular administration of the LAI composition is preferred.

In some embodiments of the first aspect of the invention, the amount of drug in the first dose and the second dose is independently selected upon each occurrence from the group consisting of about 50-150 mg, about 75-125 mg, and about 100 mg. In some embodiments, the amount of drug in the first dose and the second dose is about 100 mg.

In some embodiments of the second aspect of the invention, the amount of drug in the first dose is independently selected from the group consisting of about 200-400 mg, about 200 mg, about 300 mg, and about 400 mg, and the amount of drug in the second dose is independently selected from the group consisting of about 50-150 mg, about 75-100 mg, about 50 mg, about 75 mg, about 100 mg, about 125 mg, and about 150 mg.

In the present invention, the active ingredients letrozole and anastrozole have been selected as candidate pharmaceutical drugs for this type of prolonged release composition, because they are the first line active ingredients in the adjuvant treatment of postmenopausal women with hormone receptor-positive advanced breast cancer for whom there is no alternative therapy beyond daily administration of a tablet.

The present invention seeks to overcome the disadvantages of and/or to provide improvements over other injectable depot compositions comprising letrozole and/or anastrozole. The present invention includes one or several injectable depot composition(s), one or several implant(s) formed from said one or several injectable depot composition(s), one or several method(s) of forming said one or several implant(s), one or several kit(s) comprising components used to form said one or more injectable depot composition(s), one or several method(s) of administering letrozole and/or anastrozole by administering said one or several injectable depot composition(s), and one or several method(s) of treating one or several disease(s), condition(s), or disorder(s) that is(are) therapeutically responsive to letrozole, anastrozole or metabolite of either thereof by administering said one or several injectable depot composition(s).

The present invention employs a letrozole-containing or anastrozole-containing LAI composition suitable for forming one or more *in situ* intramuscular implants which can continuously maintain the required plasma levels of drug for hormone suppression during or for about 3 months or more, about 6 months or more, about 9 months or more, or about 12 months or more. The implant formed *in situ* with the injectable composition of the invention overcomes the majority of the drawbacks presented by current formulations based on preformed implants. It offers an alternative practical and effective therapy for the patient achieving therapeutic profiles lasting for at least 60 days, at least 120 days, at least 180 days, at least 240 days, at least 300 days, or at least one year.

This long-term hormone suppression therapy has been shown to provide a superior clinical outcome in humans compared to oral daily dosage treatment. The formulations described herein enable obtaining therapeutic levels of the drug in plasma from the start and continuously over a dosing period of at least about six months or at least about 12 months, avoiding the need for daily oral dosing regimens, thereby improving the patient's quality of life. Moreover, the dosing regimen of the invention provides a smoother plasma concentration profile of drug during the period after administration as compared to administration of a single dose of the LAI composition.

Also, the invention provides a composition, and uses thereof, that provide a sustained lower, yet still effective drug plasma levels with lower doses (compared to oral treatment on the basis of mg of drug per Kg of bodyweight) of the injectable composition. The reduced plasma levels reduce drug dose-related adverse side effects (bone mass loss, bone/joint/muscle pain, dyslipidemia) because of the lower exposure to drug due to the lower dose, while still maintaining therapeutically effective plasma levels. Moreover, the present invention provides a better safety profile (lower incidence, lower frequency of occurrence, and/or lower severity of adverse events) which positively impacts treatment duration adherence.

The present inventors have found that the dose of drug necessary for clinical efficacy is much smaller than was thought or disclosed in the art employing oral dosage forms. Thus, the compositions, and uses thereof, of the present invention provide an effective therapy for aromatase inhibition at least as early as the oral therapy from the time of administration, with much smaller doses than previous compositions, thereby providing a sustained and stable release of such low doses over long time periods (about 6 months or more, about 11 months or more, or at least 12 months) and reducing the adverse side effects.

An aspect of the invention provides a method of reducing adverse events associated with administration of certain dose of drug (letrozole, anastrozole, salt of either thereof, or metabolite of either thereof) in an extended release composition, the method comprising administering a lower dose of the drug in an injectable depot composition (or one or several respective implant(s)) of the invention, whereby said lower dose provides lower adverse events but substantially the same efficacy as a higher dose of the drug in another injectable depot composition that is not of the invention.

After administration of the LAI composition and formation of the one or more respective implant(s), said one or several implant(s) can provide(s) a pharmacokinetic performance approximately (about) as follows:

| Dose of drug administered (mg) | About 50 | About 100 |
|---|---|---|
| Daily Plasma Concentration from about 2 days after administration (ng/mL) | About 4.5 (about 0.5 to about 13) | About 8.8 (about 1.5 to about 21) |
| Cmax (ng/mL) | About 5 | About 11 |
| Tlag (h) | 0-24 or 0-48 h | 0-24 or 0-48 h |

wherein Tlag corresponds to the delay between the time of dosing and time of appearance of a measurable concentration of letrozole in the plasma. The above values are approximate and should be considered mean values.

The invention employs an injectable depot composition (and respective implant(s) thereof) and uses thereof that comprises a lower dose of drug, with respect to other injectable depot compositions not of the invention, while still providing therapeutically effective plasma levels of said drug for a period of at least about six months or more.

In some embodiments, the LAI depot composition comprises PLA having a particle size distribution as follows: particle size mass distribution with not more than 10% above 300 microns, preferably not above 250 microns, when measured by analytical sieving according to USP<786> and/or said PLA has a particle size volume distribution with a D90 not above 330 microns, preferably not above 280 microns when measured by laser diffraction analysis; and/or wherein said PLA has a particle size mass distribution where not more than 80 % of the particles have a particle size below 125 microns, when measured by analytical sieving according to USP<786> and/or said PLA has a particle size volume distribution with a D80 not below 135 microns when measured by laser diffraction analysis.

In some embodiments, the LAI comprises a sterile biodegradable thermoplastic polymer of polylactic acid (PLA), wherein said PLA has optionally been sized and has a) a particle size mass distribution with not more than 10% above 300 microns, preferably not above 250 microns, when measured by analytical sieving according to USP<786>; b) a particle size volume distribution with a D90 not above 330 microns, preferably not above 280 microns when measured by laser diffraction analysis; c) a particle size mass distribution with not more than 10% above 300 microns, preferably not above 250 microns, and where not more than 80 % of the particles have a particle size below 125 microns, when measured by analytical sieving according to USP<786>; d) a particle size mass distribution where not more than 80 % of the particles have a particle size below 125 microns, when measured by analytical sieving according to USP<786>; e) a particle size volume distribution with a D90 not above 330 microns, preferably not above 280 microns when measured by laser diffraction analysis and with a D80 not below 135 microns when measured by laser diffraction analysis; or f) has a particle size volume distribution with a D80 not below 135 microns when measured by laser diffraction analysis.

In some embodiments, the LAI composition comprises from 10 to 500 mg of letrozole and a sterile biodegradable thermoplastic polymer of polylactic acid (PLA), wherein said PLA has been optionally sized; and/or wherein said PLA has a particle size mass distribution with not more than 10% above 300 microns, preferably not above 250 microns, when measured by analytical sieving according to USP<786> and/or said PLA has a particle size volume distribution with a D90 not above 330 microns, preferably not above 280 microns when measured by laser diffraction analysis; and/or wherein said PLA has a particle size mass distribution where not more than 80 % of the particles have a particle size below 125 microns, when measured by analytical sieving according to USP<786> and/or said PLA has a particle size volume distribution with a D80 not below 135 microns when measured by laser diffraction analysis; and wherein the percentage of active agent released from the implant is ranges from about 2-30 % each 28-day interval for at least three to four intervals, preferably from about 5-25% each 28-day interval for at least three to four intervals; and/or wherein the composition releases from 0.1 to 2 milligrams of letrozole every day, preferably from 0.13 to 0.80 milligrams of letrozole every day.

In some embodiments, the implant formed from the LAI composition comprises about 50 mg of drug (letrozole and/or anastrozole) and releases an average of about 0.1 mg or more, about 0.12 mg or more, about 0.15 mg or more, about 0.1-0.15 mg, about 0.12-0.14 mg, or about 0.13 mg of drug per day for a period of at least about 337-365 days, wherein the average is based upon daily release of drug throughout entire said period. In some embodiments, the implant comprises about 100 mg of drug (letrozole and/or anastrozole) and releases an average of about 0.2 mg or more, about 0.25 mg or more, about 0.2-0.3 mg, about 0.3 mg or more, about 0.24-0.28 mg, or about 0.27 mg of drug per day for a period of at least about 337-365 days, wherein the average is based upon daily release of drug throughout entire said period.

In some embodiments, the implant releases an average of about 10-15% of its charge per 28-day interval for the first four intervals and then an average of about 2-6% of its charge per 28-day interval for the following eight to nine intervals. In some embodiments, the implant releases no more than about 60% (or no more than about 55%, or no more than about 50%) of its charge of drug within the first 112 days (four 28-day intervals) and releases the remainder of its charge of drug at a rate of about 2-6% per 28-day interval for 8 to 9 intervals (about 224 days to about 252 days). In some embodiments, the implant releases its charge of drug over a period of at least about one year, at least about 365 days, or at least about twelve to thirteen 28-day intervals. In some embodiments, the implant releases about 40-55% or about 40-50% of its charge of drug during the first 3-4 months and about 60-45% or about 60-50%, respectively, of its charge during the following 8-9 months. In some embodiments, the implant releases up to a total of about 30% of its charge during the first 28-day interval, up to a total of about 40% of its charge during the second 28-day interval, up to a total of about 50% of its charge during the third 28-day interval, up to a total of about 55% during the fourth 28-day interval, then an average of about 2-6% of its charge per 28-day interval for the following eight to nine intervals. In some embodiments, the implant releases up to a total of about 20-35% of its charge during the first 28-day interval, up to a total of about 25-40% of its charge during the second 28-day interval, up to a total of 35-50% of its charge during the third 28-day interval, up to a total of about 40-55% during the fourth 28-day interval, then an average of about 2-6% of its charge per 28-day interval for the following eight to nine intervals.

In some embodiments, the implant releases letrozole according to any of the following profiles.

| 28-day interval | Percentage of letrozole released* | Percentage of letrozole released* | Mean or average of percentage of letrozole released* |
|---|---|---|---|
| 1^{st} | 7-15 | 9-13 | About 11-12 |
| 2^{nd} | 5-12 | 6-11 | About 7.5-8.5 |
| 3^{rd} | 2-9 | 3-8 | About 3.5-7.5 |
| 4^{th} and on for each interval through final interval for at least 8 intervals | 0.5-6 | 1-6 | About 2-6 |
| 1^{st} | Up to 25 | Up to 15 | Up to 13 |
| 2^{nd} | Up to 18 | Up to 13 | Up to 11 |
| 3^{rd} | Up to 12 | Up to 10 | Up to 8 |
| 4^{th} and on for each interval through final interval for at least 8 intervals | Up to 10 | Up to 8 | Up to 6 |

| | | | |
|---|---|---|---|
| *wherein the percentage is relative to the initial charge of letrozole in the implant. | | | |

In a preferred embodiment, the LAI composition comprises a water miscible solvent, wherein the solvent is preferably dimethyl sulfoxide (DMSO).

In a preferred embodiment, the extended release (ER) composition (implant(s)) formed from the LAI composition releases up to 30% of the drug in 30 days, preferably up to 25% of the drug in 30 days; or up to 50% of the drug in 100 days, preferably in 120 days and more preferably in 130 days; or said ER composition releases up to 80% of the drug in 140 days, preferably in 180 days, more preferably in 200 days; or the ER composition releases up to 80% of the drug in 240 days, in an *in vitro* dissolution test performed with horizontal orbital motion at 50 rpm; medium: PBS pH 7.4; temperature: 37 ± 0.5 °C; analytical technique: HPLC/UV; wavelength 230 nm.

In a preferred embodiment, the LAI composition comprises from 10 to 450 mg of drug. In a preferred embodiment, the composition comprises from 30 to 90 mg or about 50 mg of drug. In a preferred embodiment, the composition comprises from 80 to 150 mg or about 100 mg of drug. In a preferred embodiment, the composition comprises from 150 to 250 mg or about 200 mg of drug. In a preferred embodiment, the composition comprises from 350 to 450 mg or about 400 mg of drug.

In preferred embodiments, the drug is letrozole.

In a preferred embodiment, the end group of the PLA is an ester group.

In a preferred embodiment, the particle size distribution of drug is such that less than 10% of the particles have a size below 20 microns, less than 10% of the particles have a size greater than 350 microns and the D50 is between 70-200 microns, when measured by laser diffraction analysis (volume distribution).

An aspect of the invention also provides a pharmaceutical kit comprising drug, PLA, and solvent for PLA, wherein the particle size distribution for said drug approximates the particle size distribution for said PLA. Said particle size distributions may be a combination of any of the particle size distributions disclosed herein.

The invention also provides a pharmaceutical kit for preparing a drug-containing LAI composition. The pharmaceutical kit can comprise a container containing drug and biodegradable polymer and a container containing pharmaceutically acceptable solvent for said biodegradable polymer. The drug, solvent, and liquid can be as defined herein.

In a preferred embodiment, the LAI composition comprises 5-40 wt. % drug, 20-40 wt. % PLA, 20-80 wt. % DMSO, with respect to the total weight of the composition before administration. Preferably, the composition comprises 15-35 wt. % drug, 25-35 wt. % PLA and 30-60 wt. % DMSO, with respect to the total weight of the composition before administration. Preferably, the LAI composition comprises 18-28 wt. % drug, 30-35 wt. % PLA and 37-52 wt. % DMSO, with respect to the total weight of the composition before administration. Alternatively, the LAI composition comprises about 23 to about 27 wt. % drug, about 28 to about 34 wt. % PLA, and about 41 to about 47 wt. % DMSO, with respect to the total weight of the composition before administration. The LAI composition may also comprise about 24 to about 26 wt. % drug, about 29 to about 33 wt. % PLA, and about 42 to about 47 wt. % DMSO, with respect to the total weight of the composition before administration.

A preferred embodiment of the of invention employs a stable LAI composition for intramuscular administration suitable for forming an *in situ* intramuscular implant comprising, preferably consisting essentially of, preferably consisting of: from 30 to 90 mg of letrozole, DMSO and a sterile biodegradable PLA, wherein the end group of the PLA is an ester group, wherein said PLA has a particle size mass distribution with not more than 10 % above 300 microns, preferably not above 250 microns, when measured by analytical sieving according to USP<786> and wherein said PLA has a particle size mass distribution where not more than 80 % of the particles have a particle size below 125 microns, when measured by analytical sieving according to USP<786> and wherein the release of the active agent from the implant is between 2 and 30 % of the active agent each 28-day interval, preferably is between 5 and 25 % of the active agent each 28-day interval; and wherein the particle size of letrozole is such that less than 10 % of the particles have a size below 20 microns, less than 10 % of the particles have a size greater than 350 microns and the D50 is between 70-200 microns, when measured by laser diffraction analysis (volume distribution); and wherein the composition comprises 15-35 wt. % letrozole, 25-35 wt. % PLA and 30-60 wt. % DMSO, with respect to the total weight of the composition before administration; and wherein the composition releases from 0.1 to 2 milligrams of letrozole every day; and wherein the stable sustained release composition provides a plasma level of letrozole between about 1 to about 40 ng/ml from about 2 days after the implant administration and continuously throughout a dosing period. The release of letrozole from this embodiment may be as otherwise specified herein.

A preferred embodiment of the invention employs a stable sustained release letrozole-containing LAI composition for intramuscular administration suitable for forming one or more *in situ* intramuscular implant(s) comprising, preferably consisting essentially of, preferably consisting of: from 80 to 150 mg of letrozole, DMSO and a sterile biodegradable PLA, wherein the end group of the PLA is an ester group, wherein said PLA has a particle size mass distribution with not more than 10% above 300 microns, preferably not above 250 microns, when measured by analytical sieving according to USP<786> and wherein said PLA has a particle size mass distribution where not more than 80% of the particles have a particle size below 125 microns, when measured by analytical sieving according to USP<786> and wherein the release of the active agent from the implant is between 2 and 30% of the active agent each 28-day interval, preferably is between 5 and 25 % of the active agent each 28-day interval; and wherein the particle size of letrozole is such that less than 10% of the particles have a size below 20 microns, less than 10% of the particles have a size greater than 350 microns and the D50 is between 70-200 microns, when measured by laser diffraction analysis (volume distribution); and wherein the composition comprises 15-35 wt. % letrozole, 25-35 wt. % PLA and 30-60 wt. % DMSO, with respect to the total weight of the composition before administration; and wherein the composition releases from about 0.1 to about 2 milligrams of letrozole every day throughout a dosing period; and wherein the stable sustained release composition provides a plasma level of letrozole between about 1 to about 40 ng/ml from about 2 days after the implant administration and continuously throughout a dosing period. The release of letrozole from this embodiment may be as otherwise specified herein.

A preferred embodiment of the invention employs a stable sustained release letrozole-containing LAI composition for intramuscular administration suitable for forming one or more *in situ* intramuscular implant(s) comprising, preferably consisting essentially of, preferably consisting of: from 150 to 250 mg of letrozole, DMSO and a sterile biodegradable PLA, wherein the end group of the PLA is an ester group, wherein said PLA has a particle size mass distribution with not more than 10% above 300 microns, preferably not above 250 microns, when measured by analytical sieving according to USP<786> and wherein said PLA has a particle size mass distribution where not more than 80% of the particles have a particle size below 125 microns, when measured by analytical sieving according to USP<786> and wherein the release of the active agent from the implant is between 2 and 30 % of the active agent each 28-day interval, preferably is between 5 and 25 % of the active agent each 28-day interval; and wherein the particle size of letrozole is such that less than 10 % of the particles have a size below 20 microns, less than 10% of the particles have a size greater than 350 microns and the D50 is between 70-200 microns, when measured by laser diffraction analysis (volume distribution); and wherein the composition comprises 15-35 wt. % letrozole, 25-35 wt. % PLA and 30-60 wt. % DMSO, with respect to the total weight of the composition before administration; and wherein the composition releases from 0.1 to 2 milligrams of letrozole every day of a dosing period; and wherein the stable sustained release composition provides a plasma level of letrozole between about 1 to about 40 ng/ml from about 2 days after the implant administration and continuously throughout a dosing period. The release of letrozole from this embodiment may be as otherwise specified herein.

A preferred embodiment of the invention employs a stable sustained release letrozole-containing composition for intramuscular administration suitable for forming one or more *in situ* intramuscular implant comprising, preferably consisting essentially of, preferably consisting of: from 350 to 450 mg of letrozole, DMSO and a sterile biodegradable PLA, wherein the end group of the PLA is an ester group, wherein said PLA has a particle size mass distribution with not more than 10 % above 300 microns, preferably not above 250 microns, when measured by analytical sieving according to USP<786> and wherein said PLA has a particle size mass distribution where not more than 80 % of the particles have a particle size below 125 microns, when measured by analytical sieving according to USP<786> and wherein the release of the active agent from the implant is in the range of about 2-30% of the active agent each 28-day interval, preferably is between 5 and 25 % of the active agent each 28-day interval; and wherein the particle size of letrozole is such that less than 10% of the particles have a size below 20 microns, less than 10% of the particles have a size greater than 350 microns and the D50 is between 70-200 microns, when measured by laser diffraction analysis (volume distribution); and wherein the composition comprises 15-35 wt. % letrozole, 25-35 wt. % PLA and 30-60 wt. % DMSO, with respect to the total weight of the composition before administration; and wherein the composition releases from 0.1 to 2 milligrams of letrozole every day of a dosing period; and wherein the stable sustained release composition provides a plasma level of letrozole between about 1 and about 40 ng/ml from about 2 days after the implant administration and continuously throughout a dosing period. The release of letrozole from this embodiment may be as otherwise specified herein.

In a preferred embodiment, the letrozole and the PLA are combined into a first container (first syringe) of the composition, and the solvent is a separate second container (second syringe) of the composition. The LAI composition is then formed by mixing the two containers.

In another aspect, the present invention employs a kit suitable for the *in situ* preparation of the LAI composition, wherein the kit comprises at least two containers or syringes, wherein the first container or syringe comprises the letrozole and the PLA, and the second container or syringe comprises the solvent, preferably DMSO. The injectable depot composition is then formed by mixing the contents of at least the first and second containers.

In a second aspect, the present invention employs a process for preparing the stable sustained release letrozole-containing LAI composition, wherein the process comprises mixing the components of the composition prior to administration. The components can be mixed as long as desired, or for 30 min or more, 30 min or less, 20 min or less, 15 in or less, preferably 10 minutes or less, and more preferably 5 minutes or less prior to administration. In a preferred embodiment, active ingredient and PLA are provided together and that combination is mixed with the solvent.

In some embodiments, the LAI composition and/or nLAI composition after preparation is a suspension. For the LAI composition, the PLA is preferably dissolved in the solvent after all components have been mixed.

In a preferred embodiment, the LAI composition is prepared by mixing the solvent, preferably DMSO, with a previous solid combination (mixture) of letrozole and PLA. In a preferred embodiment, the LAI composition is prepared (reconstituted) by first mixing the active agent with the PLA and then adding the solvent.

The invention also provides for use of the stable sustained release LAI composition for administering to a subject from about 0.1-2 mg or about 0.15-3 mg of drug every day and continuously throughout a dosing period.

In a preferred embodiment, the stable sustained release drug-containing LAI composition is used for administering to a subject, optionally a subject in need thereof, from 0.1-1.25 milligrams (mg) of letrozole every day and continuously throughout a dosing period. Preferably, the composition is used for administering a subject, optionally a subject in need thereof, from about 0.15-3 mg, 0.1 to 1.20 mg, preferably from 0.13 to 1.15 mg, more preferably from 0.13 to 1.10 mg, even more preferably from 0.13 to 0.8 mg of letrozole every day and continuously throughout a dosing period, following formation of one or more implants from said composition. The release of letrozole may be as otherwise specified herein.

In a preferred embodiment, the LAI composition provides a plasma level of letrozole between about 1 and about 40 ng/ml from about 2 days after administration and continuously throughout a dosing period. Preferably, the LAI composition forms one or more ER (extended release) implants which provide a plasma level of letrozole between about 1 and about 40 ng/ml from about 2 days after administration and continuously throughout a dosing period of at least six months, or from 6 to 12 months, or of at least 12 months. In some embodiments, the ER implant or sustained release composition of the invention provides measurable and therapeutic plasma concentrations levels of letrozole for at least about 2 years, at least about 2.5 years, at least about 2.7 years or at least about 2.8 years.

In a preferred embodiment, the stable sustained release composition forms one or more implants and provides a plasma level of letrozole in the range of about 1.5-30 ng/ml or of about 2-30 ng/ml from about 2 days after administration and continuously throughout a dosing period. Preferably, the stable sustained release composition forms one or more implants and provides a plasma level of letrozole between about 1.5 and about 30 ng/ml from about 2 days after administration and continuously throughout a dosing period of at least six months, or from 6 to 12 months, or of about 12 months or more.

In a preferred embodiment, the stable sustained release composition forms one or more implants and provides a plasma level of letrozole in the range of about 1.5-25 ng/ml or of about 2-25 ng/ml from about 2 days after administration and continuously throughout a dosing period. Preferably, the stable sustained release composition forms one or more implants and provides a plasma level of letrozole between about 1.5 and about 25 ng/ml from about 2 days after administration and continuously for at least six months, or from 6 to 12 months, or about 12 months or more.

In a preferred embodiment, the stable sustained release composition forms one or more implants and provides a plasma level of letrozole in the range of about 1.5- 20 ng/ml or of about 2-20 ng/ml from about 2 days after administration and continuously throughout a dosing period. Preferably, the stable sustained release composition forms one or more implants and provides a plasma level of letrozole between about 1.5 and about 20 ng/ml from about 2 days after administration and continuously throughout a dosing period of at least six months, or from 6 to 12 months, or of about 12 months or more.

In a preferred embodiment, the stable sustained release composition forms one or more implants and provides a plasma level of letrozole in the range of about 1.5-5 ng/ml or of about 2-15 ng/ml from about 2 days after administration and continuously throughout a dosing period. Preferably, the stable sustained release composition forms one or more implants and provides a plasma level of letrozole between about 1.5 and about 15 ng/ml from about 2 days after administration and continuously throughout a dosing period of at least six months, or from 6 to 12 months, or of about 12 months or more.

In a preferred embodiment, the stable sustained release composition provides forms one or more implants and a plasma level of letrozole in the range of about 1.5-10 ng/ml or of about 2-10 ng/ml from about 2 days after administration and continuously throughout a dosing period. Preferably, the stable sustained release composition forms one or more implants and provides a plasma level of letrozole between about 1.5 and about 10 ng/ml from about 2 days after administration and continuously throughout a dosing period of at least six months, or from 6 to 12 months, or of about 12 months or more.

In a preferred embodiment, the stable sustained release composition is used for suppressing estradiol plasma levels to less than 1 pg/ml after 4 days from the implant administration and continuously thereon throughout a dosing period. Said suppressed estradiol plasma level is maintained for at least 1 month, preferably at least 3 months, at least six months, from about 6 to about 12 months, at least 12 months, continuously from about 1 to about 12 months, continuously from about 9 to about 12 months, more preferably continuously from about 1 to about 10 months, even more preferably continuously from about 1 to about 6 months.

In a preferred embodiment, the stable sustained release composition forms one or more implants that release the drug with an immediate onset of action and continuously for at least 1 month, preferably at least 3 months, more preferably at least 6 months, even more preferably at least 12 months. In a preferred embodiment, the stable sustained release composition forms one or more implants that release the drug with an immediate onset of action and continuously from about 1 to about 12 months or from about 9 to about 12 months, from about 1 to about 10 months, or from about 1 to about 6 months.

In some embodiments, the stable sustained release composition forms one or more implants and releases the drug with an immediate onset of action and continuously throughout a dosing period of about 3 to about 6 months or of about 3 to about 9 months or of about 3 to about 12 months.

In a preferred embodiment, the stable sustained release composition is formed from an injectable intramuscular depot and sterile composition suitable for forming one or more *in situ* solid, semisolid or gel implant(s) in a subject. In a preferred embodiment, the stable sustained release composition is a single implant.

The composition(s) and implant(s) of the invention are preferably used in humans.

In another aspect, the present invention relates to the use of a letrozole-containing LAI composition suitable for forming an *in situ* intramuscular implant comprising a sterile biodegradable thermoplastic polymer of polylactic acid (PLA), for administering to a subject from about 0.1 -2 mg or about -.15-3 mg of drug every day throughout a dosing period of. Preferably, the use is for administering to a subject from about 0.13 to about 1.25 milligrams every day throughout a dosing period of.

In a preferred embodiment, the LAI composition forms one or more implants and provides a plasma level of letrozole about 1 to about 40 ng/ml after 2 days from administration and continuously throughout a dosing period. More preferably, the composition forms one or more implants and provides a plasma level of letrozole from about 1.5 to about 30 ng/ml after 2 days from administration and continuously throughout a dosing period.

In a preferred embodiment of this aspect, the LAI composition (the one or more implants formed therefrom) is used for suppressing estradiol plasma levels to less than about 1 pg/ml after 4 days or less from the implant administration and continuously throughout a dosing period.

In a preferred embodiment of this aspect, the LAI composition is used for aromatase inhibition, preferably in humans. In a preferred embodiment of this aspect, the composition is used for treating breast cancer. In a preferred embodiment of this aspect, the composition is used for adjuvant treatment of postmenopausal women with hormone receptor positive early breast cancer or for extended adjuvant treatment of postmenopausal women with early breast cancer who have received prior standard adjuvant tamoxifen therapy, or for first and second-line treatment of postmenopausal women with hormone receptor positive or unknown advanced breast cancer or any combination thereof.

The invention also provides a method of treating a disease, disorder or condition that is therapeutically responsive to nonsteroidal inhibitor of aromatase, which can be selected from the group consisting of letrozole, anastrozole, and a metabolite of either thereof. The disease, disorder or condition may be selected from the group consisting of hormonally-responsive breast cancer, local or metastatic breast cancer that is hormone receptor positive or has an unknown receptor status in postmenopausal women, hormone receptor positive early breast cancer in postmenopausal women, early breast cancer in postmenopausal women who have received prior standard adjuvant tamoxifen therapy, advanced breast cancer in postmenopausal women with disease progression following tamoxifen therapy, ER-negative disease in subjects who did not respond to previous tamoxifen therapy, gynecomastia, and endometriosis. The breast cancer can be early-stage breast cancer or advanced-stage (late-stage) breast cancer. The composition may be administered as part of adjuvant therapy, first-line therapy, or second-line therapy. The composition may also be used to induce ovulation in a female subject or to promote spermatogenesis in a male subject (e.g. one suffering from nonobstructive azoospermia).

In some embodiments, the subject in need thereof is a woman with breast cancer, optionally a postmenopausal woman with breast cancer. In some embodiments, the subject is not also being concurrently administered tamoxifen.

The dosing regimen of the invention may be practiced before and/or after surgery in a subject.

Another aspect of the present employs a drug-containing LAI composition suitable for intramuscular administration and comprising a sterile biodegradable thermoplastic polymer of polylactic acid (PLA), wherein the LAI composition releases from about 0.1-2 mg of drug every day and continuously throughout a dosing period.

In a preferred embodiment of this aspect, the LAI composition releases the drug with an immediate onset of action and continuously for at least 1 month, preferably at least about 3 months or more, more preferably at least about 6 months or more, even more preferably at least about 12 months or more. In a preferred embodiment of this aspect, the LAI composition releases the drug with an immediate onset of action and continuously for at least 1 month, at least about 2 months or more, at least about 3 months or more, at least about 4 months or more, at least about 5 months or more, at least about 6 months or more, at least about 7 months or more, at least about 8 months or more, at least about 9 months or more, at least about 10 months or more, at least about 11 months or more, or at least about 12 months or more. In a preferred embodiment, the LAI composition releases the drug with an immediate onset of action and continuously for at least about 12 months or more. In another preferred embodiment, the LAI composition releases the drug with an immediate onset of action and continuously between about 3 and about 6 months.

In a preferred embodiment of this aspect, the LAI composition is a sterile injectable intramuscular depot composition suitable for forming an *in situ* solid, semisolid or a gel implant in a body.

This long-term sustained hormone suppression therapy has been shown to provide a superior clinical outcome in humans compared to oral daily dosage treatment. Also, sustained lower effective letrozole plasma level with lower doses (compared to oral treatment) reduce the adverse side effects (bone mass loss, bone/joint/muscle pain, dyslipidemia) due to lower exposure to drug. Moreover, the present invention provides a better safety profile which positively impact treatment duration adherence.

The invention includes all combinations of the aspects, embodiments, sub-embodiments and clauses disclosed herein. Other features, advantages and embodiments of the invention will become apparent to those skilled in the art by the following description, accompanying examples and appended claims.

### BRIEF DESCRIPTION OF THE FIGURES

The following drawings are part of the present specification and are included to further demonstrate certain aspects of the invention. The invention may be better understood by reference to one or more of these drawings in combination with the detailed description of the specific embodiments presented herein.
Figure 1 depicts a chart of estradiol (E2) plasma levels (pg/ml) observed versus time after administration of FEMARA^{®} or the LAI composition (Letrozole ISM). More rapid and sustained estradiol suppression were achieved using substantially lower doses of the composition of the invention (and/or substantially lower plasma levels of letrozole) as compared to FEMARA^{®} dose and plasma level of letrozole.
Figure 2 depicts a chart of letrozole plasma levels (ng/ml) observed versus time after administration of FEMARA^{®} or the LAI composition (Letrozole ISM). The composition of the invention provided a substantially longer sustained letrozole plasma levels. Letrozole plasma levels (ng/ml) observed after administering FEMARA^{®}.
Figure 3 depicts a chart of cumulative percentage of released letrozole versus time (d) in an *in vitro* dissolution test from a composition comprising PLA having a particle size mass distribution where more than 10 % of the particles have a particle size of 300 microns or more, when measured by analytical sieving according to USP<786>.
Figure 4 depicts a chart of cumulative percentage of released letrozole versus time from a composition of the invention in an *in vitro* dissolution test.
Figure 5 depicts a chart of cumulative percentage of released letrozole versus in an *in vitro* dissolution test from a composition comprising PLA having a particle size mass distribution wherein at least 80 % of the particles have a particle size of 125 microns or less, when measured by analytical sieving according to USP<786>.
Figures 6A and 6B depict chart of estradiol (E2) plasma levels (pg/ml) observed versus time after administration of FEMARA^{®} or the LAI composition (Letrozole ISM comprising 50 mg of letrozole). The inset depicts an expanded view of the first 15 days.
Figures 7A and 7B depict charts of estradiol (E2) plasma levels (pg/ml) observed versus time after administration of FEMARA^{®} or the LAI composition (Letrozole ISM comprising 100 mg of letrozole). The inset depicts an expanded view of the first 15 days.
Figure 8 depicts a chart of letrozole plasma levels (ng/ml) observed versus time after administration of FEMARA^{®} or LAI compositions (Letrozole ISM 50 mg or 100 mg).
Figure 9 depicts a chart of letrozole average and intersubject variability 95^{th} and 5^{th} percentile plasma levels (ng/ml), observed versus time after intramuscular administration of a single 100 mg dose of LAI.
Figure 10 depicts a chart of the average and intersubject variability 95^{th} and 5^{th} percentile level of E2 suppression (%) observed versus time after intramuscular administration of a single 100 mg dose of LAI.
Figure 11 depicts a chart of the average and intersubject variability 95^{th} and 5^{th} percentile level of E2 suppression (%) observed versus time after intramuscular administration of a first 100 mg dose of LAI composition and, about 8 weeks after administration of the first dose, intramuscular administration of a second 100 mg dose of LAI composition. This is one of the preferred embodiments of the invention.
Figure 12 depicts three exemplary embodiments of the dosing regimens of the invention, wherein "DP1" denotes "dosing period 1", "DP2" denotes "dosing period 2", "DP3" denotes "dosing period 3", "TP" denotes "treatment period", "1^{st} LAI" denotes "first dose of LAI composition", "2^{nd} LAI" denotes "second dose of LAI composition", "3^{rd} LAI" denotes "third dose of LAI composition"; "HD" denotes "higher dose", and "LD" denotes "lower dose".

### DETAILED DESCRIPTION OF THE INVENTION

As used herein, the terms "disease, disorder or condition that is therapeutically responsive to nonsteroidal inhibitor of aromatase" excludes any disease, disorder or condition that is not therapeutically responsive to nonsteroidal inhibitor of aromatase. The therapeutic responsive of said disease, disorder or condition to nonsteroidal inhibitor of aromatase is determined by administering one or more compositions of the invention to a subject in need thereof, wherein the dose of drug (via the composition) is administered as defined herein to provide one or more implants that release said nonsteroidal inhibitor of aromatase for the period of time as defined herein to provide the plasma levels of said drug or metabolite thereof as defined herein. A clinician then determines whether said disease, disorder or condition responds therapeutically, meaning the clinician determines whether the subject in need has experienced the desired clinical benefit(s). For example, when treating cancer, a therapeutic response may include slowing or reversing the progression of the cancer or might even include remission of the cancer. When treating gynecomastia, a therapeutic response may include reduction in breast size or slowing down the enlargement of the breast. A clinician familiar with nonsteroidal inhibitors of aromatase will be able to follow methods and conventions of the art to determine whether said disease, disorder or condition is therapeutically responsive to said nonsteroidal inhibitor of aromatase.

As used herein, the term "dosing period" refers to the period from administration of LAI composition to termination of release of drug from a corresponding one or more implants. As used herein, the term "treatment period" refers to the period during which a subject receives dose(s) of the injectable depot composition. A treatment period may comprise one or more dosing periods during which time the subject receives one or more corresponding doses. The treatment period preferably comprises at least the first dosing period and the delayed and overlapping second dosing period. The treatment period can last about one to ten years, about two years or more, about three years or more, about five years or more, about seven years or more, or about ten years or more. The treatment period includes at least administration of the first two doses of LAI composition and can further include about annual administration of LAI composition.

As used herein, the terms "letrozole" and "anastrozole" refers to the non-salt and salt forms thereof. The term "active ingredient", "active agent", or "drug" refers to a therapeutically active compound, as well as any, derivatives thereof, prodrugs thereof, and pharmaceutically acceptable salts, hydrates and solvates of said compound, derivative(s), and prodrug(s). In the present invention, the active agent (drug) is letrozole and/or anastrozole. The preferred drug is letrozole.

The term "stable sustained release composition" generally refers to the one or more implant(s), which together provide a dose of drug during a dosing period.

The term "stable" as used herein refers to a pharmaceutical composition comprising letrozole wherein the total content of impurities originating from the decomposition of letrozole does not exceed 5% area, preferably 3% area, more preferably 2% area and most preferably 1% area determined by liquid chromatography (HPLC) at 230 nm if such a composition is stored for 2 months at 40 °C and 75% relative humidity (RH).

Use of a nLAI composition as a first dose of drug in the dosing regimen of the invention is optional. As used herein, a non-long-acting injectable (nLAI) composition is an injectable composition that excludes an extended (sustained, controlled) release polymer. The nLAI comprises drug and at least one pharmaceutically acceptable liquid excipient, which is preferably aqueous. Additional pharmaceutically acceptable excipients are optionally included in the nLAI.

The drug may be completely suspended or partially dissolved/partially suspended in the liquid excipient of the nLAI or LAI compositions. In preferred embodiments, at least part of the drug is suspended in particulate form in aqueous liquid excipient or water miscible liquid excipient. In some embodiments, the liquid excipient (which may also be referred to herein as the solvent) comprises DMSO or NMP (N-methylpyrrolidone). Suitable liquid excipients are non-toxic, biocompatible and appropriate for parenteral injection and preferably are biocompatible so as not to cause severe tissue irritation or necrosis at the injection site. The liquid excipient is preferably classified as class II or III, and more preferably class III, according to ICH Guidelines. For the formation of the *in-situ* implant, the liquid excipient should preferably diffuse quickly from the polymeric solution towards surrounding tissues when is exposed to physiological fluids. Consequently, the liquid excipient is preferably water miscible and more preferably with a dipole moment about 3.9-4.3 D at 25°C. The preferred liquid excipients are DMSO (dimethylsulfoxide) and PEG (poly(ethylene glycol, such as PEG having an average molecular weight in the range of about 200, about 300 or about 400). Grades of PEG that are liquid at ambient temperature (20-30° C) may be used. The most preferred liquid excipient is DMSO. The invention may also employ a LAI composition having a combination of two or more of these liquid excipients.

As used herein, a long-acting injectable (LAI) composition of the invention comprises drug, at least one liquid excipient, and at least one biodegradable extended (controlled, sustained) release polymer. The polymer can be a homopolymer or a copolymer. The drug may be completely dissolved, partially dissolved/partially suspended, or completely suspended as particles in the LAI before administration. The polymer may be completely dissolved, partially dissolved/partially suspended, or completely suspended as particles in the LAI before administration. The drug and polymer may be present together as drug-containing particles suspended in the at least one liquid excipient. The composition may further comprise one or more other pharmaceutical excipients.

In preferred embodiments, the LAI composition comprises drug, at least one biodegradable extended release polymer, and at least one solvent (liquid excipient) for the polymer. Particularly preferred embodiments of the LAI composition comprise drug, PLA, and solvent for PLA. The amount of solvent in the LAI is preferably sufficient to dissolve the PLA. The drug may be partially or completely suspended in particulate form in the polymeric solution (PLA dissolved in the solvent) or solvent. In some embodiments, about 50% or more of the drug is suspended in particulate form. In other embodiments, about 60% or more, about 70% or more, about 80% or more, or about 90% or more of the drug is suspended in particulate form.

As used herein, "sized" PLA ("PLA that has been sized") is PLA that has been processed to provide a particular particle size distribution as described herein. Sizing of the PLA is optional. Methods of sizing PLA, include by way of example and without limitation, milling, comminuting, sieving, classifying, grinding, impacting, homogenizing, sonicating, ultrasonicating, dry milling, wet milling, cryogenic milling or other such processes known in the art of particle size reduction.

When the PLA particle size is measured by analytical sieving according to USP<786>, the amplitude is 0.65 mm and the shaking time is 5 minutes. When the PLA particle size is measured by laser diffraction analysis, the particle size is determined by wet dispersion method. No sample pre-treatment was applied. The sample was directly added into the dispersion medium (water). Dispersion mechanism was stirring at 3000 rpm.

The PLA polymer is selected from free acid (not end-capped; uncapped) or end-capped (e.g. alkyl esters such as lauryl ester, methyl ester, etc., referred to herein as PLA-e) terminal carboxylic polylactide with polymer. The PLA polymer can be a poly(L-lactic acid) polymer, poly(D,L-lactic acid) polymer, poly (D-lactic acid) or a copolymer of those polymers. Polymers that are end-capped with esters (as opposed to the free carboxylic acid) demonstrate longer degradation half-lives; even so, uncapped polymers having carboxylic acid terminal groups also exhibit the particle size-related improved performance. Suitable grades of PLA are commercially available from Uhde Inventa-Fischer (Berlin, DE), NatureWorks LLC (Blair, NE, USA), Plastic Ingenuity (Cross Plains, WI, USA), Toyobo, Dai Nippon Printing Co., Mitsui Chemicals, Shimadzu, NEC, Toyota (Japan), PURAC Biomaterials, Hycail (The Netherlands), Galactic (Belgium), Cereplast (U.S.A.), FkuR, Biomer, Stanelco, Inventa-Fischer (Germany), Snamprogetti (China), Boehringer Ingelheim (RESOMER^{®} grades; Ingelheim Am Rhein, DE), Evonik Industries (RESOMER^{®} grades; Essen, DE), ALKERMES (Dublin, Ireland) or SIGMA ALDRICH (ST. Louis, MO). In some embodiments, the PLA is end-capped with an alkyl alcohol to form an ester end group moiety.

In some embodiments, the PLA polymer exhibits an inherent or intrinsic viscosity in the range of about 0.16-0.60 dl/g, or about 0.20-0.50 dl/g as measured in chloroform at 25°C at a concentration of 0.1% wt/v with a Ubbelohde size 0c glass capillary viscometer or as measured in chloroform at 30°C and at a concentration of 0.5% wt/v with a size 25 Cannon-Fenske glass capillary viscometer. The inherent viscosity can be as measured before or after beta irradiation, if such irradiation is employed.

As used herein, the term "polymeric solution" is taken to mean the fluid composition comprising a combination of the solvent and the polymer dissolved therein. In some embodiments, at least 80%, at least 90%, at least 95%, at least 99% or all of the polymer is dissolved in the solvent. The LAI composition comprises (or consists essentially or consists of) solvent, PLA polymer and drug. Accordingly, the LAI composition comprises (or consists essentially or consists of) polymeric solution and drug.

In some embodiments, the polymeric solution has a minimum viscosity of about 0.8 Pa.s, although this may preferably be around 1 Pa.s. The viscosity can be about 4 Pa.s or less, about 3 Pa.s or less, about 2 Pa.s or less, about 1.8 Pa.s or less. The viscosity of the polymer solution can range from about 0.7 to about 4 Pa.s, about 0.7 to about 3 Pa.s, about 0.7 to about 2 Pa.s, about 0.8 to about 1.8 Pa.s or about 1 to about 1.8 Pa.s. The viscosity of polymeric solutions in DMSO is measured at 25 °C. If not otherwise specified, the viscosity value of the polymeric solution or the injectable composition is given in Pa.s units.

In some embodiments, the weight ratio of DMSO to PLA is about 1:1 to about 2.3:1, about 1.2:1 to about 1.8:1, about 1.3:1 to about 1.5:1, or about 1.4:1.

In some embodiments, the weight ratio of DMSO to drug is in the range of about 0.5 to about 3.7, about 1:1 to about 3:1, about 1.5:1 to about 2:1, about 1.7:1 to about 1.8:1, or about 1.75:1.

In some embodiments, the weight ratio of polymer solution to drug is about 1:1 to about 5.7:1, about, 2.3:1 to 4:1, about 2.8:1 to about 3.2:1, or about 3:1.

In some embodiments, the weight ratio of PLA to drug is about 0.6:1 to about 2.8:1, about 1.0 to 2.0, about 1.1:1 to 1.6:1, about 1.1:1 to about 1.4:1, about 1.1:1 to about 1.35:1, about 1.1:1 to about 1.3:1, about 1.2:1 to about 1.3:1, or about 1.25:1.

In some embodiments, the drug is partially dissolved or completely undissolved in the polymeric solution. In some embodiments, ≤5%, ≤10%, ≤20% wt of the drug is dissolved in the solvent or polymeric solution to form the injectable composition. In some embodiments, >0%, ≥0.5%, ≥1%, ≥5%, or ≥ 10% wt. of the drug is dissolved in the solvent or polymeric solution to form the injectable composition.

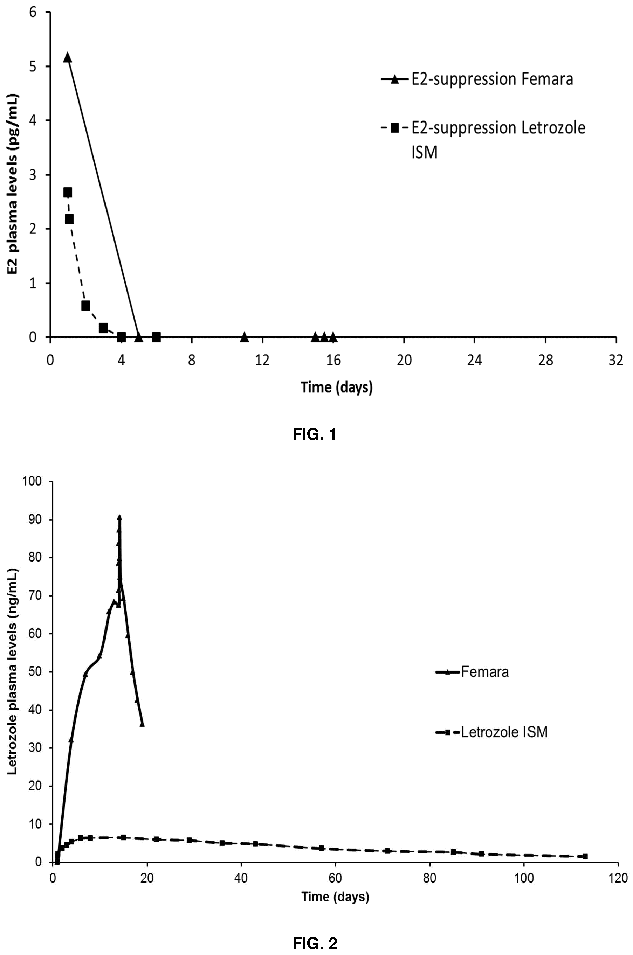

The expression "immediate onset of action" as used herein means that the estrogen plasma level suppression achieved by the composition of the invention is at least as early as the one achieved by oral therapy with FEMARA^{®}, namely at day 4. For example, FEMARA^{®} (oral) and a composition of the invention (Letrozole ISM) were compared by administering a dose of each to human subjects. The results (depicted in FIG. 1) demonstrate that no estradiol (E2) can be detected, meaning E2 is below the detection limit. Importantly, however, a lower dose of letrozole was administered as Letrozole ISM than as FEMARA^{®}. Accordingly, the LAI composition is able to reduce the plasma level of E2 to less than 1.0 pg/mL, less than 0.8 pg/mL, less than 0.5 pg/mL, less than 0.3 pg/mL, or less than 0.1 pg/mL within about four days after administration and continuously throughout a dosing period.

The performance of FEMARA^{®} and a composition of the invention (Letrozole ISM) was compared (FIG. 2) in terms of plasma concentration achieved after administration of a dose to human subject. The FEMARA^{®} composition provided extremely high plasma concentrations of letrozole, which can then be associated with much higher incidence or severity of adverse events. On the other hand, the composition of the invention provided a) no burst release of letrozole; b) substantially longer sustained letrozole plasma levels of letrozole; and c) therapeutically effective plasma levels of letrozole for about 16 weeks (about 4 months) or more.

The relative impact of particle size distribution of PLA was determined by comparing the in vitro dissolution profile (drug release profile) for various extended release (ER) compositions formed from injectable depot compositions comprising PLA of different particle size distributions.

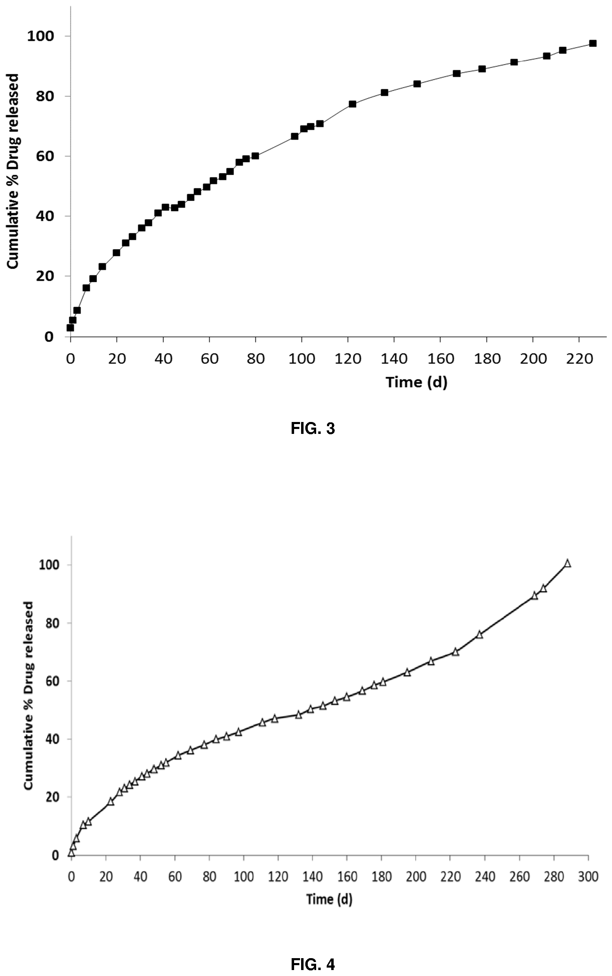

The impact of the relative content of larger PLA particles was determined. FIG. 3 depicts a chart of cumulative percentage of released letrozole versus time (d) in an *in vitro* dissolution test from a composition (not according to the invention) comprising PLA having a particle size mass distribution where more than 10 % of the particles have a particle size of 300 microns or more, when measured by analytical sieving according to USP<786>. The PLA used in the composition of FIG. 3 had a particle size mass distribution where 18.1 % of the particles were bigger than 300 microns and 28.3 % of the particles were smaller than 125 microns, measured by analytical sieving according to USP<786>. Said PLA had a particle size volume distribution with a D90 of 421 microns and a D80 of 324 microns when measured by laser diffraction analysis.

FIG. 4 depicts a chart of cumulative percentage of released letrozole versus time from a LAI composition in an *in vitro* dissolution test. The PLA used had a particle size mass distribution where 0.8 % of the particles were bigger than 300 microns and 58.5 % of the particles were smaller than 125 microns, measured by analytical sieving according to USP<786>. Said PLA had a particle size volume distribution with a D90 of 214 microns and a D80 of 170 microns when measured by laser diffraction analysis. The sustained release was satisfactory and was unexpectedly about two months longer than that provided by the PLA not according to the invention (FIG. 3).

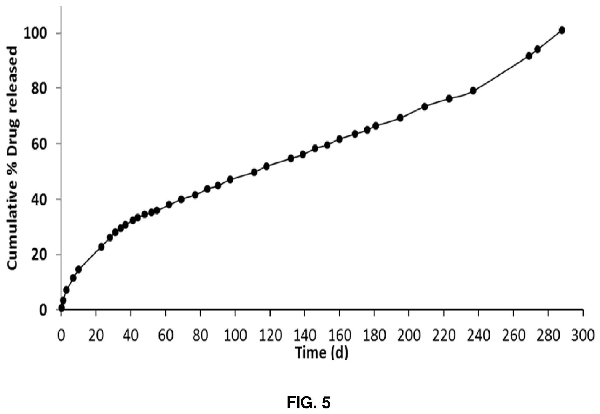

The impact, upon drug dissolution, of the relative content of smaller PLA particles was determined. FIG. 5 depicts a chart of cumulative percentage of released letrozole versus in an *in vitro* dissolution test from a LAI composition comprising PLA having a particle size mass distribution wherein at least 80 % of the particles have a particle size of 125 microns or less, when measured by analytical sieving according to USP<786>. The PLA used in the composition had a particle size mass distribution where 1.6 % of the particles were bigger than 300 microns and 88.8 % of the particles were smaller than 125 microns, measured by analytical sieving according to USP<786>. Said PLA had a particle size volume distribution with a D90 of 155 microns and a D80 of 124 microns when measured by laser diffraction analysis. The implant provided satisfactory sustained release results; even though, it formed hard agglomerates during initial mixing of the DMSO, PLA and drug.

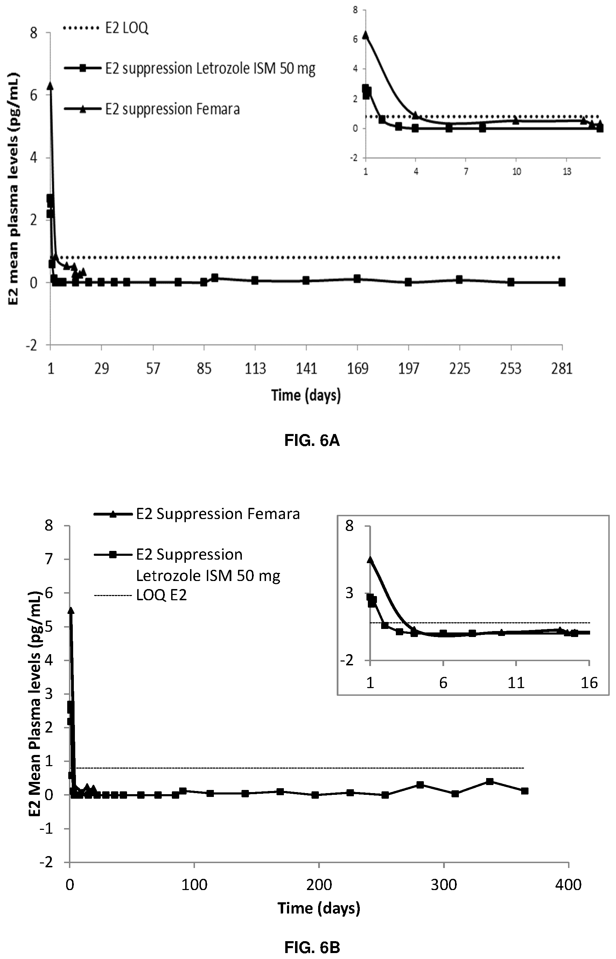

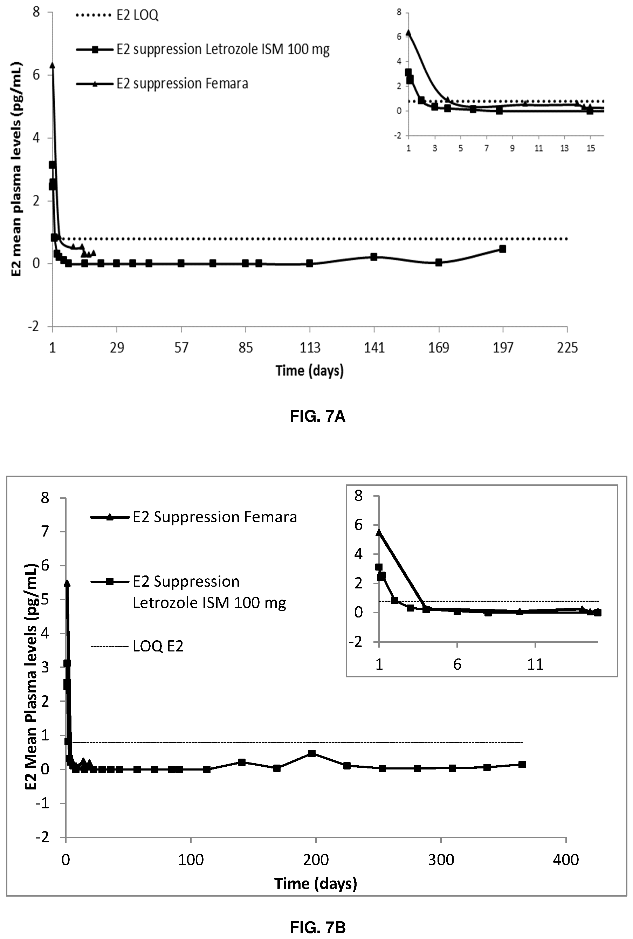

FIGS. 6A and 6B depict chart of estradiol (E2) plasma levels (pg/ml) observed versus time after oral administration of FEMARA^{®} (2.5 mg once daily for 14 days) or intramuscular administration of the LAI composition (Letrozole ISM; one injection comprising 50 mg of letrozole) according to Example 3 (Cohort 1). The inset depicts an expanded view of the first 15 days. FIGS. 7A and 7B depict charts of estradiol (E2) plasma levels (pg/ml) observed versus time after oral administration of FEMARA^{®} (2.5 mg once daily for 14 days) or intramuscular administration of the composition of the invention (Letrozole ISM; one injection comprising 100 mg of letrozole) according to Example 3 (Cohort 2). The inset depicts an expanded view of the first 15 days. Plasma estrogen levels decreased rapidly from baseline following administration of letrozole. The hormones decreased to stable levels below 1 pg/mL at approximately 4 days post-Femara^{®}/Letrozole ISM treatment (FIGS. 6A, 6b, 7A and 7B). The sustained suppression of estrogen levels was maintained for at least about 365 days (or at least about 6 months, at least about 9 months, at least about 10 months, at least about 11 months, or at least about 12 months) for Letrozole ISM 50 mg and 100 mg. There were no apparent differences in the extent of hormonal level decrease between Letrozole ISM 50 mg and 100 mg. It is very surprising that a 50 mg dose injectable composition was as effective as a 100 mg dose injectable composition in terms of E2 suppression.

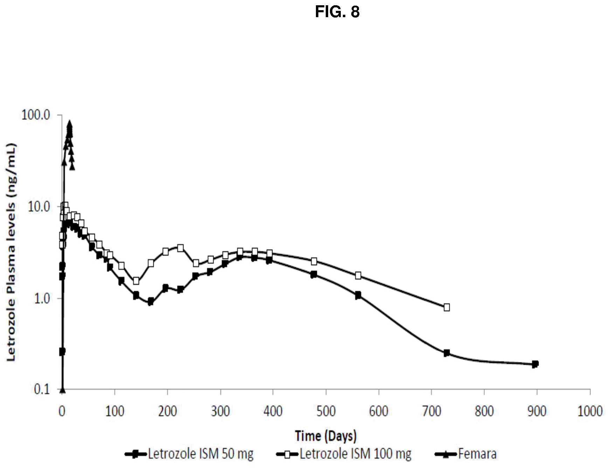

FIG. 8 depicts a chart of letrozole plasma levels (ng/ml) observed versus time after administration of FEMARA^{®} (2.5 mg once daily for 14 days) or the compositions of the invention (Letrozole ISM 50 mg or 100 mg) corresponding to FIGS. 6 and 7. Letrozole mean maximum exposure concentrations observed for FEMARA^{®} at steady state (2.5 mg QD for 14 days) were approximately 12-fold and 8-fold greater than that observed for Letrozole 50 mg and 100 mg doses, respectively. The much higher plasma concentration provided by FEMARA^{®} would provide corresponding higher severity and/or incidence of adverse events as compared to the much lower and still therapeutically effective plasma concentration provided by the composition(s) of the invention.

By overlaying the charts of FIGS. 6A, 6B, 7A, and 7B, the 100 mg LAI composition has twice the dose of letrozole as the 50 mg injectable composition, both compositions are effective at maintaining the plasma levels of E1 (estrone) and E2 (estradiol) below the LOQ, the level of quantitation, for at least about 365 or more days.

Accordingly, the LAI composition(s) provide(s) therapeutically effective plasma levels of letrozole for at least 6 months, at least 7 months, at least 8 months, at least 9 months, at least 10 months, at least 11 months, or at least 12 months, while concomitantly providing reduced adverse events.

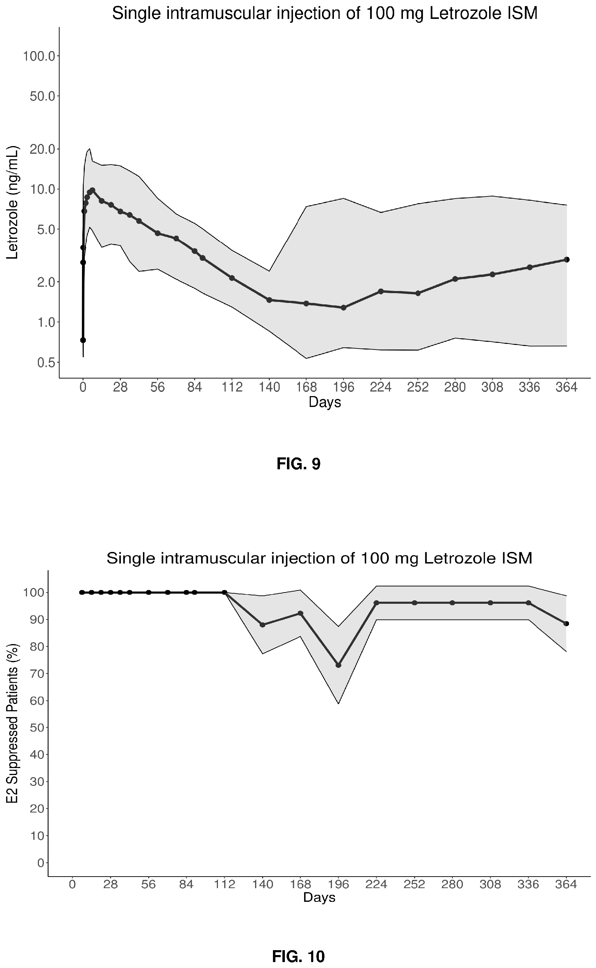

FIG. 9 depicts a graph of the time dependent change in average letrozole plasma concentration (ng/mL) following intramuscular administration of a single 100 mg dose of LAI composition comprising letrozole, PLA, and DMSO. The shaded area above and below the line represents observed intersubject 95^{th} and 5^{th} percentile variability in the plasma concentration.

FIG. 10 depicts the percent suppression of E2 corresponding to the plasma profile of FIG. 9. The level of E2 suppression exhibits an undesirable (disadvantageous) substantial decrease (change) between about 112-224 days after single administration of the LAI composition (100 mg single dose). The consequence of this change is that a subject receiving the LAI composition may or will likely not receive the full therapeutic benefit of the drug during that time period and may be at higher risk of cancer progression during the period of decreased E2 suppression.

The present inventors developed a combination dosing regimen that overcomes the disadvantage observed with sole administration of a single dose of the LAI composition. They determined that first intramuscular administration of a LAI composition followed by second intramuscular administration of LAI composition, after a delay period of about 6-10 weeks (first aspect of the invention) or about 50-54 weeks (second aspect of the invention), essentially eliminated the undesired period of reduced E2 suppression typically following administration of a single LAI composition.

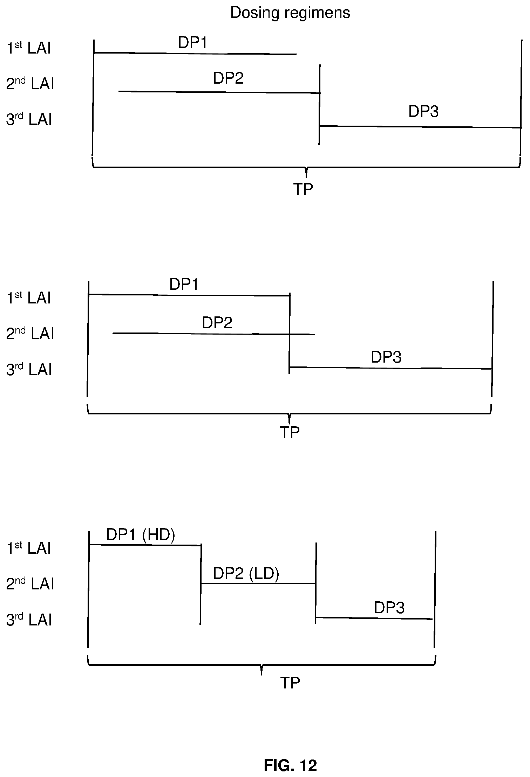

FIG. 12 depicts three exemplary dosing regimens of the invention. According to the first embodiment of the first aspect of the invention, a first dose of LAI (1^{st} LAI) is administered, thus starting a first dosing period (DP1). After a delay period of about 6-10 weeks or about 8 weeks, a second dose of LAI (2^{nd} LAI) is administered, thus starting a second dosing period (DP2). DP1 and DP2 each last about 11-13 months or about 12 months. A third dose of LAI (3^{rd} LAI) is administered toward the end of DP2, i.e. administered within about 4 weeks before to about 4 weeks after the end of DP2, thus starting a third dosing period (DP3). Additional subsequent annual administrations of LAI composition are contemplated, thereby further extending the overall TP.

According to the second embodiment of the first aspect of the invention (FIG. 12), a first dose of LAI (1^{st} LAI) is administered, thus starting a first dosing period (DP1). After a delay period of about 6-10 weeks or about 8 weeks, a second dose of LAI (2^{nd} LAI) is administered, thus starting a second dosing period (DP2). DP1 and DP2 each last about 11-13 months or about 12 months. A third dose of LAI (3^{rd} LAI) is administered toward the end of DP1, i.e. administered within about 4 weeks before to about 4 weeks after the end of DP1. Additional subsequent annual administrations of LAI composition are contemplated, thereby further extending the overall TP.

According to a first embodiment of the second aspect of the invention (FIG. 12), a first dose of LAI (1^{st} LAI) is administered, thus starting a first dosing period (DP1- HD). This is the higher dose dosing period. After a delay period of about 50-54 weeks or about 52 weeks, a second dose of LAI (2^{nd} LAI) is administered, thus starting a second dosing period (DP2-LD). This is the lower dose dosing period. DP1-HD and DP2-LD each last about 11-13 months or about 12 months. A third dose of LAI (3^{rd} LAI) is administered toward the end of DP2-LD, i.e. administered within about 4 weeks before to about 4 weeks after the end of DP2-LD. Additional subsequent annual administrations of LAI composition are contemplated, thereby further extending the overall TP. The dose for the 3^{rd} LAI dose can be greater than, less than, or about the same as the dose for the 2^{nd} LAI dose.

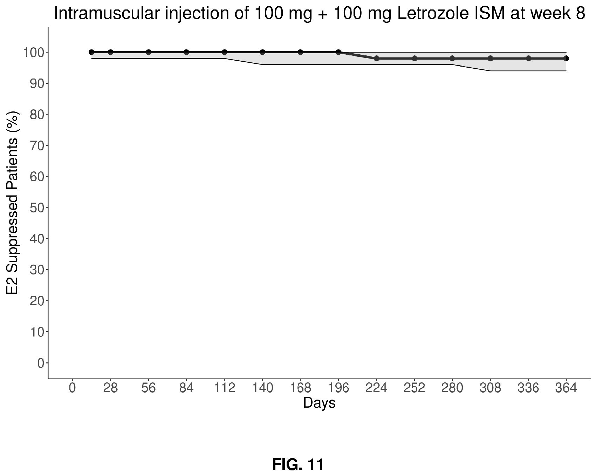

According to the first aspect of the invention, a first dose of LAI composition (PLA, DMSO, letrozole) comprising about 100 mg of drug, in particular letrozole, was administered intramuscularly. At about 8 weeks after administration of the first dose of LAI composition, a second dose of LAI composition (PLA, DMSO, letrozole) comprising about 100 mg of drug, was administered intramuscularly. The resulting profile for extent (%) of E2 suppression is depicted in FIG. 11. The period of decreased E2 suppression at 112-224 days was essentially eliminated. As a result, patients being treated with drug will receive the full therapeutic benefit of the drug throughout the TP.

If used in a dosing regimen, the nLAI composition may comprise the same solvent (pharmaceutically acceptable liquid excipient) or different solvent than that present in the LAI composition.

Either composition may further comprise one or more pharmaceutically acceptable excipients selected from the group consisting of water miscible solvent, surfactant, buffer, solubilizer, chelating agent, preservative, antioxidant, adsorbents, acidifying agent, alkalizing agent, antifoaming agent, buffering agent, colorant, electrolyte, salt, stabilizer, tonicity modifier, or oil. Suitable solvents are listed in the "International Conference on Harmonisation of Technical Requirements for Registration of Pharmaceuticals for Human Use (ICH) guidance for industry Q3C Impurities: Residual Solvents" (1997), which makes recommendations as to what amounts of residual solvents are considered safe in pharmaceuticals.

The phrase "pharmaceutically acceptable" is employed herein to refer to those compounds, materials, compositions, and/or dosage forms which are, within the scope of sound medical judgment, suitable for use in contact with tissues of human beings and animals and without excessive toxicity, irritation, allergic response, or any other problem or complication, commensurate with a reasonable benefit/risk ratio.

The invention provides a method of treating a disease, disorder or condition that is therapeutically responsive to nonsteroidal inhibitor of aromatase. Said nonsteroidal inhibitor can be letrozole, anastrozole, or metabolite of either thereof. Exemplary diseases, disorders or conditions include, for example: a) adjuvant treatment (treatment following surgery with or without radiation) of postmenopausal women with hormone receptor-positive early breast cancer; b) metastasis in both pre and post-menopausal women; c) precocious puberty or children with pubertal gynecomastia; d) reducing estrogens, including estradiol, in men; e) hormonally-responsive breast cancer after surgery; f) ovarian stimulation; g) promote spermatogenesis in male patients suffering from nonobstructive azoospermia; h) endometriosis; i) cancer that is estrogen hormone receptor-positive or sensitive (non-small cell lung cancer, uterine leiomyomas, etc.); j) infertility in women with polycystic ovarian syndrome; k) ovarian cancer; l) breast cancer that is estrogen hormone receptor positive or sensitive; j) priming for in vitro maturation cycles; k) preoperative treatment with letrozole in premenopausal women undergoing laparoscopic myomectomy of large uterine myomas; l) short stature in peripubertal boys; m) unexplained infertility or infertility with unknown or uncertain etiology; n) idiopathic central precocious puberty in boys.

In some embodiments, the LAI composition is formed by mixing solvent, PLA polymer and drug to form the injectable depot composition as defined herein. The solvent, PLA and drug may be in one, two, three or more containers. In one embodiment, PLA and drug are in a container and solvent is in a separate container and the contents of the containers are mixed to form the composition. In another embodiment, PLA, drug, and solvent are in separate containers, and the contents of the containers are mixed to form the composition. In another embodiment, PLA and solvent are in a container, and drug is in a separate container, and the contents of the containers are mixed to form the composition. The container(s) may be part of a pharmaceutical kit. Accordingly, the invention also provides a pharmaceutical kit comprising the one or more containers, wherein the content of the container(s) are as described herein.

The method may further comprise the step of forming the injectable composition before administration thereof. The method may further comprise the step of providing a kit comprising containers with the ingredients of the injectable composition. The composition can be administered every about 30 days, about 45 days, about 60 days, about 90 days, about 120 days, or 150 days, or about every month, about every two months, about every three months, about every four months, about every five months, about every six months, about every nine months, about every ten months, about every eleven months, about every twelve months, or as often as needed for as many times needed to ameliorate the disease, disorder or condition. Combinations of the dosing regimens herein are contemplated. The composition provides therapeutic plasma levels for a period of at least 1 month, at least 2 months, at least 3 months, at least 4 months, at least 5 months, at least 6 months, at least 9 months, at least 10 months, at least 11 months, or at least twelve months following administration of a dose of said composition.

The injectable composition can be administered to muscle tissue, adipose tissue, peritoneum, or below the skin. Intramuscular administration is preferred. In some embodiments, the composition is administered to the gluteal and/or deltoid muscles. The composition can also be administered to the quadriceps muscle group. A dose can be administered to a single muscular site or can be divided into two or more portions and administered to two or more muscular sites of a subject. For example, a first portion of a dose can be administered to a first section of gluteal muscle and a second portion of the dose can be administered to a second section of gluteal muscle of a subject. A single-body implant will form at each injection site. Such a mode of administration within a same day is considered to be administration of a single dose with a single dosing period. Alternatively, administration can be modified such that there is one point of needle entry into the subject but more than one injection site below the skin, which can be achieved by making a first penetration into the skin and muscle and administering a portion of a dose, then partially withdrawing and redirecting the needle into another section of muscle, while maintaining the tip of the needle beneath the skin, and then injecting another portion of the dose into this other section of muscle. Such a mode of administration is still considered to be administration of a single dose within a single dosing period.

A dose of the injectable composition typical comprises about 30 to about 90 mg of letrozole, about 50-100 mg of letrozole, about 80 to about 150 mg of letrozole, about 150 to about 250 mg of letrozole, about 250 to about 350 mg of letrozole, about 350 to about 450 mg of letrozole, about 20 to about 500 mg of letrozole, about 50 mg of letrozole, about 75 mg of letrozole, about 100 mg of letrozole, about 200 mg of letrozole, or about 400 mg of letrozole. Anastrozole can be used in place of letrozole.

Following administration to a subject, the preferred LAI composition will form one or more implants (preferably a single implant) in said subject.

Administration of a single dose is typically considered that amount of injectable composition administered to a subject within a period of up to 24 hours, up to 12 hours, up to 6 hours, up to 3 hours, up to one hour, up to 30 min, up to 15 min or up to 5 min.

In another embodiment, the injectable composition is sterile as a finished product. In another embodiment, the biocompatible polymer is sterilized previously to its aseptic filling process, preferably by irradiation (such as beta irradiation) or by another process, e.g. filtration.

The implant of the invention can provide substantially improved plasma levels of drug when compared to another injectable formulation (not according to the invention) containing the same drug when administered on an equivalent dose basis.

The invention also provides an extended release implant comprising PLA and drug (which is letrozole, anastrozole, or salt of either thereof, or metabolite of either thereof), wherein a) the weight ratio of PLA to drug is about 0.6:1 to about 2.8:1, about 1.0 to 2.0, about 1.1:1 to 1.6:1, about 1.1:1 to about 1.4:1, about 1.1:1 to about 1.35:1, about 1.1:1 to about 1.3:1, about 1.2:1 to about 1.3:1, or about 1.25:1; b) the implant has been prepared from a LAI composition comprising i) 15-35 wt. % letrozole, 25-35 wt. % PLA, and 30-60 wt. % DMSO; ii) 18-28 wt. % letrozole, 30-35 wt. % PLA, and 37-52 wt. % DMSO; iii) about 23 to about 27 wt. % letrozole, about 28 to about 34 wt. % PLA, and about 41 to about 47 wt. % DMSO; or iv) about 24 to about 26 wt. % letrozole, about 29 to about 33 wt. % PLA, and about 42 to about 47 wt. % DMSO, wherein the weight percentages are with respect to the total weight of the composition before formation of the implant. When administered to a subject, the implant releases from about 0.1 to about 2 milligrams of letrozole daily and provides a plasma level of letrozole in the range of about 1.5-40 ng/ml or about 2-40 ng/ml from about 2 days after administration and continuously throughout a dosing period. Prior to formation of the implant, the particle size distribution of the PLA in the composition is as defined herein according to the invention. The PLA is optionally sized before being included in the composition. The implant may be used in a method of administering or a method of treating as defined herein.

The invention also provides a method of forming an implant, the method comprising a) mixing DMSO, PLA and drug to form a LAI depot composition; and b) administering the composition to a subject. The content and characteristics/properties of the ingredients are as defined herein. The particle size distribution of the PLA, prior to implant formation, is as defined herein. The in vivo and in vitro performance of the implant is as defined herein.

All values disclosed herein may have standard technical measure error (standard deviation) of ± 10%. The term "about" is intended to mean ±10%, ±5%, ±2.5% or ±1% relative to a specified value, i.e. "about 20%" means 20±2%, 20±1%, 20±0.5% or 20±0.25%.

In view of the above description and the examples below, one of ordinary skill in the art will be able to practice the invention as claimed without undue experimentation. The foregoing will be better understood with reference to the following examples that detail certain procedures for the preparation of embodiments of the present invention. All references made to these examples are for the purposes of illustration. The following examples should not be considered exhaustive, but merely illustrative of only a few of the many embodiments contemplated by the present invention.

### Example 1

### LAI Compositions

The following formulations are prepared.

A ready-to-use formulation can be prepared, for example, and included in a syringe ready for use for intramuscular injection. The same formulation may form part, for example, of a kit of two syringes, one male and one female or two male syringes linked by a connector in which the solution of PLA in DMSO is in one syringe and the letrozole is in solid form in a second syringe. Similarly, the final composition can be obtained by, for example, maintaining one syringe with the PLA and letrozole in solid state and the solvent (DMSO) in a second syringe.
Formulation 1:

| Ingredient | Amount (mg, % wt) |
|---|---|
| Lactic acid polymer (ester terminal group) of intrinsic viscosity of 0.3 dl/g, irradiated as raw material at 10 kGy. | 55.20 (35.8%) |
| Dimethyl sulfoxide | 82.80 (53.7%) |
| Letrozole | 16.20 (10.5%) |

The weight ratio of DMSO to PLA is about 1.5:1. The weight ratio of DMSO to drug is about 5.11:1. The weight ratio of polymeric solution to drug is about 8.52:1. The weight ratio of PLA to drug is about 3.41:1.
Formulation 2: formulation with letrozole in suspension

| Ingredient | Amount (mg, % wt) |
|---|---|
| Lactic acid polymer (ester terminal group) of intrinsic viscosity of 0.3 dl/g, irradiated as raw material at 10 kGy. | 38.80 (30%) |
| Dimethyl sulfoxide | 58.30 (45%) |
| Letrozole | 32.40 (25%) |

Letrozole particle size in formulation 2 was characterized by the technique of laser ray diffraction (Malvern Mastersizer 2000, suspended in water until obscuration of 9.41%) and had the following distribution (in % volume): d(0.1) = 38.21 µm, d(0.5) = 141.35 µm and d(0.9) = 312.13 µm.

The weight ratio of DMSO to PLA is about 1.5:1. The weight ratio of DMSO to drug is about 1.8:1. The weight ratio of polymeric solution to drug is about 3:1. The weight ratio of PLA to drug is about 1.2:1.
Formulation 3:

| Ingredient | Amount (mg) |
|---|---|
| Lactic acid polymer (carboxylic terminal group) of intrinsic viscosity of 0.3 dl/g, irradiated as raw material at 10 kGy. | 38.80 |
| Dimethyl sulfoxide | 58.30 |
| Letrozole | 32.40 |

The weight ratio of DMSO to PLA is about 1.5:1. The weight ratio of DMSO to drug is about 1.8:1. The weight ratio of polymeric solution to drug is about 3:1. The weight ratio of PLA to drug is about 1.2:1.
Formulation 4:

| Ingredient | Amount (mg) |
|---|---|
| Lactic acid polymer (ester terminal group) of intrinsic viscosity of 0.3 dl/g, irradiated as raw material at 10 kGy. | 38.80 |
| Dimethyl sulfoxide | 58.30 |
| Letrozole | 32.40 |

The weight ratio of DMSO to PLA is about 1.5:1. The weight ratio of DMSO to drug is about 1.8:1. The weight ratio of polymeric solution to drug is about 3:1. The weight ratio of PLA to drug is about 1.2:1.
Formulation 5:

| Ingredient | Amount (mg, % wt) |
|---|---|
| Lactic acid polymer (carboxylic terminal group) of intrinsic viscosity of 0.3 dl/g, irradiated as raw material at 10 kGy. | 107.6 (31.1%) |
| Dimethyl sulfoxide | 151.7 (43.9%) |
| Letrozole | 86.5 (25%) |

The weight ratio of DMSO to PLA is about 1.41:1. The weight ratio of DMSO to drug is about 1.75:1. The weight ratio of polymeric solution to drug is about 3:1. The weight ratio of PLA to drug is about 1.25:1.
Formulation 6:

| Ingredient | Amount (mg) |
|---|---|
| Lactic acid polymer (ester terminal group) of intrinsic viscosity of 0.3 dl/g, irradiated as raw material at 10 kGy. | 107.6 |
| Dimethyl sulfoxide | 151.7 |
| Letrozole | 86.5 |

The weight ratio of DMSO to PLA is about 1.41:1. The weight ratio of DMSO to drug is about 1.75:1. The weight ratio of polymeric solution to drug is about 3:1. The weight ratio of PLA to drug is about 1.25:1.
Formulations 7 to 12:

| Formulation | 7 | 8 | 9 | 10 | 11 | 12 |
|---|---|---|---|---|---|---|
| Ingredient | Amount (% wt) | Amount (% wt) | Amount (% wt) | Amount (% wt) | Amount (% wt) | Amount (% wt) |
| Lactic acid polymer (PLA) terminating in a carboxylic group | 28-36 | 15-42.5 | 29.6-32.7 | | | |
| Lactic acid polymer (PLA) ester terminal group | | | | 15-42.5 | 28-36 | 29.6-32.7 |
| Dimethyl sulfoxide | 38.5-52 | 25-59.5 | 42.2-45.6 | 25-59.5 | 38.5-52 | 42.2-45.6 |
| Letrozole | 20-30 | 15-50 | 24-26 | 15-50 | 20-30 | 24-26 |

For some preferred compositions, the % w/w in the composition of the active agent was between 20.0 and 27.0 %. The % w/w in the composition of the PLA was between 20.0 and 50.0 %. The % w/w in the composition of the solvent was between 23.0 and 60.0 %. The compositions were mixed in a syringe to form suitable implants.

Different types of PLA were used for these compositions:
1. PLA with particle size mass distribution where more than 10 % of the particles had a particle size of 300 microns or above when measured by analytical sieving according to USP<786>;
2. PLA with particle size volume distribution with D90 above 330 microns when measured by laser diffraction analysis;
3. PLA with particle size mass distribution where no more than 10 % of the particles had a particle size above 300 microns and no more than 80 % of the particles have a particle size below 125 microns when measured by analytical sieving according to USP<786>;
4. PLA with particle size volume distribution with D90 not above 330 microns and D80 not below 135 microns when measured by laser diffraction analysis;
5. PLA with particle size mass distribution where more than 80 % of the particles have a particle size below 125 microns, when measured by analytical sieving according to USP<786>;
6. PLA with particle size volume distribution with D80 below 135 microns when measured by laser diffraction analysis.

Compositions of the invention were prepared first by dry mixing the active agent with the PLA and then adding the solvent, preferably DMSO to dissolve the PLA and have a suspension of the active agent. The reconstitution process is to be carried out immediately prior to injection, and the time for preparation does not exceed 15 minutes, preferably 10 minutes, more preferably 5 minutes, before the IM composition is administered.

The implants prepared this way were used for the following dissolution test: horizontal orbital motion at 50 rpm; medium: PBS pH 7.4.; temperature: 37 ± 0.5 °C; analytical technique HPLC/UV; wavelength 230 nm.

It was observed that for PLAs 1 and 2, the release was not as satisfactory sustained as desired when preferred time of preparation was applied (FIG. 3). However, for PLAs 3 and 4, the sustained release was satisfactory (FIG. 4).

When PLAs 5 and 6 were used, big and hard agglomerates formed, and the composition could not be prepared within the preferred time of no more than 15 minutes. Thus, this composition was not considered clinically suitable. In any case, the implant was assayed, and sustained release resulted satisfactory (FIG. 5).

### Example 2

### Particle size determination

### Analytical sieving according to USP<786>

The PLA particle size mass distribution was determined by sieve stack technique using the following sizes: 425 > 355 > 300 > 250 > 212 > 180 > 150 > 125 > 106 > 75. The amplitude was 0.65 mm and the shaking time 5 minutes.

### Laser light diffraction

The PLA particle size distribution is expressed as volume distribution and was determined by laser diffraction technique by wet dispersion method. No sample pre-treatment was applied. The sample was directly added into the dispersion medium (water). Dispersion mechanism was stirring at 3000 rpm and the sample was stabilized for 30 seconds before measuring.

### Example 3

### Clinical Data

### Preliminary Phase I results

Preliminary results suggest that sustained long-term hormone suppression therapy (HT) may obtain a superior clinical outcome in breast cancer compared to an oral daily dosage treatment.

Early discontinuation and non-adherence to HT are common and associated with increased mortality - improved treatment compliance with Letrozole ISM^{®} has potential to enhance treatment.

Sustained lower effective doses (compared to oral treatment) could reduce adverse side effects (bone mass loss, bone/joint/muscle pain, dyslipidemia) due to lower exposure to drug.

Better safety profile has potential to positively impact treatment duration adherence.

### Phase I results

This is a Phase I, open label, dose escalation study designed to evaluate the pharmacokinetics, safety, and tolerability of single intramuscular injections of Letrozole ISM at different strengths in approximately 120 voluntary healthy postmenopausal women. The study has four arms:
Experimental: Cohort 1: Letrozole ISM 50 mg: 14 oral doses of 2.5 mg FEMARA^{®} (once daily) + single IM injection of 50 mg Letrozole ISM.
Experimental: Cohort 2: Letrozole ISM 100 mg: 14 oral doses of 2.5 mg FEMARA^{®} (once daily) + single IM injection of 100 mg Letrozole ISM.
Experimental: Cohort 3: Letrozole ISM 200 mg: 14 oral doses of 2.5 mg FEMARA^{®} (once daily) + single IM injection of 200 mg Letrozole ISM.
Experimental: Cohort 4: Letrozole ISM 400 mg: 14 oral doses of 2.5 mg FEMARA^{®} (once daily) + single IM injection of 400 mg Letrozole ISM.

The objective of this study is to assess the pharmacokinetic profile of single ascending doses of Letrozole ISM, and secondly, to evaluate safety and tolerability of single ascending doses of Letrozole ISM, measure estrogen levels, and characterize oral letrozole pharmacokinetic profile to be used in subsequent comparison to Letrozole ISM.

The study is carried out in healthy post-menopausal women who satisfy inclusion and exclusion criteria. The study design includes a screening period and 2 treatment periods. Treatment Period 1 comprises of 14 oral dose administrations of 2.5 mg Femara^{®}. Treatment Period 2 comprises of a single IM dose of 50, 100, 200 and 400 mg Letrozole ISM. The total planned study duration is 71 weeks, approximately.

### Inclusion Criteria:

The inclusion/exclusion criteria for the 120 participants are the following:
- Healthy post-menopausal women, ≥ 18 and ≤ 75 years of age, who have achieved complete menopause, either natural or surgical, and amenorrhea, and have not been on hormone replacement therapy in the last 3 months.
- Post-menopausal subjects should have absence of menses for 1 year, and oophorectomized subjects should have absence of menses for at least 6 weeks. For oophorectomized subjects and subjects who have had a hysterectomy, a surgical pathology report documenting the absence of malignant disease is required. In addition, for oophorectomized subjects an operative report documenting bilateral oophorectomy is required.
- Baseline follicle-stimulating hormone (FSH) and 17β-estradiol plasma levels should be consistent with the post-menopausal status of the subject (FSH ≥ 40 mIU/mL; 17β-estradiol ≤ 31 pg/mL), confirmed at least 48 hours prior to dosing.
- Weight of ≥ 50 kg and a BMI ≥ 19 and ≤ 39 kg/m2.
- Subjects will be in good health, as determined by medical history, physical examination, vital signs assessments (pulse rate, systolic and diastolic blood pressure, and temperature), clinical laboratory evaluations, and 12-lead ECG. Minor deviations outside the reference ranges will be acceptable, if deemed not clinically significant by the investigator.
- Subjects who have not had a mammogram within the last 12 months (documentation required) must be willing to have one performed.
- Subjects with an intact uterus and cervix who have not had a Papanicolaou (pap) smear test within the last 6 months (documentation required) must be willing to have one performed.
- Subjects will have given their written informed consent to participate in the study and to abide by the study restrictions.
- Subjects should be able to communicate with clinic staff.

### Exclusion Criteria:

- Subjects who have a history of allergy or hypersensitivity to letrozole or any of the inactive ingredients in the last 3 months.
- Subjects who have a history of galactose intolerance, severe hereditary lactase deficiency glucose-galactose malabsorption.
- Subjects who have used estrogen or progesterone hormone replacement therapy, thyroid replacement therapy, oral contraceptives, androgens, luteinizing hormone (LH) releasing hormone analogs, prolactin inhibitors, or antiandrogens within 3 months prior to Screening.
- Subjects who have regularly taken foods or food supplements that contain high levels of Isoflavinoids, including soybean, soymilk, soynuts, chickpeas, alfalfa, fava beans, kudzu, miso and tofu in the 14 days prior to dosing (Treatment Period 1). The investigator and medical monitor will determine on a case-by-case basis if a subject who intakes food or food supplements containing Isoflavinoids is eligible to participate in the study.
- Subjects who have used:
   ∘ Any medications including St. John's wort, known to be potent or moderate inducers of CYP P450 3A4 in the 3 weeks prior to dosing (Treatment Period 1).
   ∘ Any medications or products known to be potent or moderate inhibitors of CYP P450 3A4 (e.g. grapefruit juice) in the 7 days prior to dosing on Treatment Period 1.
   ∘ Any prescribed preparations within 14 days prior to dosing (Treatment Period 1), unless in the opinion of the investigator (or designee) the medication will not interfere with the study procedures or compromise safety.
   ∘ Any non-prescribed systemic or topical medications within 7 days of dosing (Treatment Period 1) unless in the opinion of the investigator (or designee) the medication will not interfere with the study procedures or compromise safety. Vitamins and minerals including the use of calcium and/or vitamin D for osteoporosis prevention are allowed.
- Subjects who have been diagnosed with osteoporosis (previously or results from screening DEXA for this study with a T score < -2.5). Subjects with osteopenia (with the T-score between -1 and -2.5) will be allowed to participate in this study.
- Subjects who are not on a stable dose of long- or short-acting bisphosphonates therapy for at least 3 months prior to Screening.
- Subjects who are on raloxifene therapy.
- Subjects who have an abnormality in heart rate, blood pressure, or temperature at Screening and prior to first dose (Treatment Period 1) that in the opinion of the investigator increases the risk of participating in the study. Resting SBP must be ≤ 150mmHg and resting DBP ≤ 95 mmHg.
- Subjects who have an abnormality in the 12-lead ECG at Screening and prior to first dose (Treatment Period 1) that in the opinion of the Investigator increases the risk of participating in the study.
- Subjects who have any clinically significant abnormal physical examination finding.
- Subjects who have any clinically significant abnormal laboratory safety findings at Screening or Check-in, upon repeat testing, as determined by the investigator (1 repeat assessment is acceptable).
- Subjects who have ALT or AST >1.5 × ULN. For subjects with elevated total bilirubin, direct and indirect bilirubin will be evaluated.
- Subjects with elevated cholesterol or triglyceride levels above the ULN must be determined by the Investigator to be not clinically significant.
- Subjects who have relevant diseases or clinically significant abnormal relevant findings at Screening, as determined by medical history, physical examination, laboratory, ECG, DEXA, and breast and pelvic examination.
- Subjects who have history of any significant chronic disease, such as but not limited to: thrombotic disorders, coronary artery or cerebrovascular disease, liver, kidney or gallbladder dysfunction/disorder(s), diabetes or any other endocrine disease, estrogen dependent neoplasia, post-menopausal uterine bleeding, or endometrial hyperplasia. Subjects with cholecystectomy will be permitted if no medical sequelae post-surgery.
- Subjects who have a history of cancer within the past 5 years with the exception of non-melanoma skin cancer.
- Subjects who have a history of drug-dependence, and recent history of alcoholism or abuse of alcohol.
- Subjects who have a positive result for hepatitis B surface antigen (HBsAg), hepatitis B core antibody, hepatitis C antibody, or human immunodeficiency virus (HIV) antibodies.
- Subjects with a positive drugs of abuse screen or alcohol breath test at Screening (urine will be screened for the presence of the following: amphetamine, barbiturates, benzodiazepines, cannabinoid, cocaine, opiates, phencyclidine, and methadone).
- Subjects with a history of, or difficulty of, access to veins for venipuncture.
- Subjects who have donated blood in the 30 days prior to first dose (Treatment Period 1).
- Subjects who have received blood products within 2 months prior to Screening.
- Subjects who have received a drug in research or have participated in other clinical trials within 30 days, or 5 half-lives (whichever is longer) prior to dosing (Treatment Period 1).
- Subjects who have previously taken part in or have withdrawn from this study. (Subjects who have been screened for but not included in a cohort or subjects who dropped out from screening in a previous cohort for non-medical reasons may be eligible to be included in subsequent cohorts.)
- Any other unspecified reason that, in the opinion of the investigator (or designee) or Sponsor, makes the subject unsuitable for enrolment.

### PK results

Letrozole plasma concentrations have been analyzed up to Day 897 and Day 729 following a single intramuscular (IM) injection of Letrozole ISM 50 mg and 100 mg, respectively. Moreover, plasma levels of letrozole has also been analyzed for up to 897 and 729 days. The majority of subjects had sustained quantifiable letrozole plasma concentrations up to the last sampling time points reported in both groups (FIG. 8). Dose-normalized peak exposure (Cmax/D) is comparable between both dose strength of Letrozole ISM. Accordingly, the implant or sustained release composition of the invention provides measurable and therapeutic plasma concentrations levels of letrozole for at least about 2 years, at least about 2.5 years, at least about 2.7 years or at least about 2.8 years.

### Example 4

### Comparison of dosing regimens

Two LAI compositions, each containing letrozole (about 100 mg strength), PLA and DMSO were provided. The compositions were administered as follows.

### Single LAI administration

Subjects were administered the LAI compositions according to the following schedule: First day-an amount (0.4 ml) of LAI composition comprising a dose of 100 mg of letrozole was administered intramuscularly. The plasma concentration of letrozole (ng/mL; FIG. 9) and the extent of E2 suppression (%; FIG. 10) were determined on a daily or periodic basis.

### Sequential LAI dosing regimen

Subjects were administered the compositions according to the following schedule: First day- a first amount (0.4 mL) of LAI composition comprising a dose of 100 mg of letrozole was administered intramuscularly; and at the end of eight weeks from said first day- a second amount (0.4 mL) of LAI composition comprising a dose of 100 mg of letrozole was administered intramuscularly. After each administration, the extent of E2 suppression (%; FIG. 11) was determined on a daily or periodic basis.

### Example 5

### Additional LAI compositions

The following formulations are prepared.
Formulation 13.

| Ingredient | Amount (mg, % wt) |
|---|---|
| Lactic acid polymer | 124 (31%) |
| Dimethyl sulfoxide | 176 (44%) |
| Letrozole | 100 (25%) |

Formulation 14.

| Ingredient | Amount (mg, % wt) |
|---|---|
| Lactic acid polymer | 124 (31%) |
| Dimethyl sulfoxide | 176 (44%) |
| Anastrozole | 100 (25%) |

Formulation 15.

| Ingredient | Amount (mg, % wt) |
|---|---|
| Lactic acid polymer | 96 (32%) |
| Dimethyl sulfoxide | 129 (43%) |
| Letrozole | 75 (25%) |

Formulation 16.

| Ingredient | Amount (mg, % wt) |
|---|---|
| Lactic acid polymer | 96 (32%) |
| Dimethyl sulfoxide | 129 (43%) |
| Anastrozole | 75 (25%) |

Formulation 17.

| Ingredient | Amount (mg, % wt) |
|---|---|
| Lactic acid polymer | 155.5 (31.1%) |
| Dimethyl sulfoxide | 219.5 (43.9%) |
| Letrozole | 125 (25%) |

Formulation 18.

| Ingredient | Amount (mg, % wt) |
|---|---|
| Lactic acid polymer | 155.5 (31.1%) |
| Dimethyl sulfoxide | 219.5 (43.9%) |
| Anastrozole | 125 (25%) |

### Example 6

### Dosing regimen of the second aspect

The LAI formulations are prepared as described above and administered as follows. Subjects meeting the selection criteria of Example 3 are administered at least two sequential doses of LAI: a first higher dose LAI-HD (having 200-400 mg of letrozole or anastrozole) and after a delay period a subsequent lower dose LAI-LD (having 75-100 mg of letrozole or anastrozole, respectively. Each LAI composition has a dosing period of about 50-54 weeks.

Each subject is administered intramuscularly a dose of the LAI-HD. After about 50-54 weeks or about 52 weeks, each subject is administered intramuscularly a dose of the LAI-LD.

Optionally, on an as needed basis, a subject is administered another dose of LAI about 50-54 weeks or about 52 weeks after administration of the LAI-LD. The subject may continue to receive annual doses of the LAI composition for as many years as clinically necessary to provide the desired clinical benefit or therapeutic result.

## Claims

1. A long-acting injectable composition comprising polylactic acid (PLA), DMSO, and at least one aromatase inhibitor drug selected from the group consisting of letrozole, anastrozole, and combinations thereof, **characterized in that** the particle size distribution of the PLA is as follows:
- a mass particle size distribution with no more than 10% of particles larger than 300 microns and no more than 80% of particles smaller than 125 microns, when measured by analytical sieving according to USP<786>; and
- a particle size distribution by volume in which less than 10% of the particles are smaller than 20 microns, less than 10% of the particles are larger than 350 microns, the D50 is between 70 and 200 microns, the D80 is not less than 135 microns, and the D90 is not greater than 330 microns, when measured by laser diffraction analysis.

2. The composition according to claim 1, **characterized in that** the particle size distribution of the PLA is as follows:
- a mass particle size distribution with no more than 10% of particles larger than 250 microns when measured by analytical sieving according to USP<786>; and
- a particle size distribution by volume with a D90 of no more than 280 microns when measured by laser diffraction analysis.

3. The composition according to claims 1 or 2, **characterized in that** it comprises 15-35% by weight of drug, 25-35% by weight of PLA, and 30-60% by weight of DMSO, where the weight percentages are based on the total weight of said composition.

4. The composition according to any of claims 1-3, **characterized in that** it comprises 18-28% by weight of drug, 30-35% by weight of PLA, and 37-52% by weight of DMSO, wherein the weight percentages are based on the total weight of said composition.

5. The composition according to any of claims 1-4, **characterized in that** it comprises 23-27% by weight of the drug, 28-34% by weight of PLA, and 41-47% by weight of DMSO, wherein the weight percentages are based on the total weight of said composition.

6. The composition according to any of claims 1 to 5, **characterized in that** the particle size distribution of said aromatase inhibitor drug is approximately the same as the particle size distribution of said PLA.

7. The composition according to any of claims 1 to 6, **characterized in that** the drug is present in said composition as a suspension, and the PLA is dissolved in the solvent in said composition.

8. The composition according to any of claims 1 to 7, **characterized in that** the PLA is terminated with ester end groups.

9. The composition according to any one of claims 1 to 8, **characterised in that** the weight ratio of DMSO to PLA is from 1.3:1 to 1.5:1, or is approximately 1.4:1.

10. The composition according to claim 9, **characterised in that** the weight ratio of DMSO to drug is in the range of 1.5:1 to 2:1, 1.7:1 to 1.8:1, or is approximately 1.75:1.

11. The composition according to any one of the preceding claims 1-10, wherein the amount of drug in said composition is selected from the group consisting of 10-450 mg, 30-90 mg, approximately 50 mg, 50-100 mg, 80-150 mg, 75-125 mg, approximately 75 mg, approximately 100 mg, 150-250 mg, approximately 200 mg, around 350-450 mg, and around 400 mg.

12. The composition according to any one of the preceding claims 1-11, for use in the treatment of a subject in need of such treatment.

13. The composition for use as claimed in claim 12 in the treatment of a disease, condition or disorder responsive to an aromatase inhibitor in a subject, comprising administering to the subject the composition of any one of claims 1 to 11.

14. The composition for use according to claims 12 or 13, comprising a) administering intramuscularly to a subject an amount of a long-acting injectable composition according to any of the preceding claims 1 to 11, comprising a first dose of an aromatase inhibitor; and b) subsequently, after a delay period, administering intramuscularly a quantity of a long-acting injectable composition comprising a second dose of an aromatase inhibitor.

15. The composition for use according to claim 14, wherein the delay period is approximately 6 weeks or more after administering said first dose of the composition.

16. The composition for use according to claim 15, wherein the delay period is selected from: approximately 6-10 weeks, approximately 48-60 weeks or approximately 50-54 weeks.

17. The composition for use according to any of claims 12 to 16, which further comprises administering a third dose of said composition approximately 1 year after said first dose or said second dose.

18. The composition for use according to any of claims 12 to 17, comprising the administration of an additional dose of said composition annually over a total treatment period of approximately three years or more, approximately 5 years or more, approximately 7 years or more, or approximately 10 years.

19. The composition for use according to any of claims 12 to 18, wherein the amount of drug in said first dose is greater than the amount of drug in said second dose.

20. The composition for use according to any of claims 12 to 18, wherein the amount of drug in said first dose is approximately equal to or less than the amount of drug in said second dose.

21. A composition for use according to any of claims 12 to 19, wherein the first dose comprises approximately 200-400 mg of drug, and the second dose comprises approximately 75-100 mg of drug.

22. A composition for use according to any of claims 12 to 21, wherein said disease, disorder or condition is selected from the group consisting of: a) adjuvant treatment (treatment following surgery, with or without radiotherapy) of postmenopausal women with early-stage, hormone-receptor-positive breast cancer; b) metastases in pre- and postmenopausal women; c) precocious puberty or children with pubertal gynaecomastia; d) reducing oestrogens, including oestradiol, in men; e) hormone-sensitive breast cancer following surgery; f) ovarian stimulation; g) promoting spermatogenesis in male patients suffering from non-obstructive azoospermia; h) endometriosis; i) oestrogen receptor-positive or oestrogen-sensitive cancer (non-small cell lung cancer, uterine leiomyomas); j) infertility in women with polycystic ovary syndrome; k) ovarian cancer; l) oestrogen receptor-positive or oestrogen-sensitive breast cancer; m) priming for in vitro maturation cycles; n) pre-operative treatment with letrozole in premenopausal women undergoing laparoscopic myomectomy for large uterine leiomyomas; o) short stature in peripubertal children; p) unexplained infertility or infertility of unknown or uncertain aetiology; q) idiopathic central precocious puberty in children; r) gynaecomastia; (s) endometriosis; (t) ER-negative disease in subjects who have not responded to prior tamoxifen therapy; (u) early-stage breast cancer in postmenopausal women who have previously received standard adjuvant tamoxifen therapy; and (v) advanced breast cancer in postmenopausal women with disease progression following tamoxifen treatment.

23. An intramuscular *in situ* implant comprising the composition according to any one of claims 1 to 11.

24. The intramuscular implant of claim 23, wherein the drug is letrozole and the implant releases from 0.1 to 2 mg of letrozole daily following administration to a subject in need thereof.
